# EUROPEAN PATENT SPECIFICATION

(11) **EP 1 235 918 B1**
(45) Date of publication and mention of the grant of the patent: **21.03.2007**
(21) Application number: 00982261.0
(22) Date of filing: 29.11.2000
(51) Int. Cl.: C12N 15/81, C12N 15/31, C12Q 1/68

(54) **METHODS FOR ALTERING THE EXPRESSION OF HYPHAL-SPECIFIC GENES**
VERFAHREN ZUM ÄNDERN DER EXPRESSION VON HYPHEN-SPEZIFISCHEN GENEN
PROCEDES DE MODIFICATION DE L'EXPRESSION DES GENES SPECIFIQUES DE L'HYPHE

(30) Priority: 29.11.1999 US 167672 P
(43) Date of publication of application: 04.09.2002
(73) Proprietor: Trustees of Dartmouth College, Hanover, NH 03755-1404 (US)
(72) Inventor: Sundstrom, Paula, Lebanon, NH 03766 (US)
(74) Representative: Becker, Philippe
(86) International application number: PCT/US2000/032464
(87) International publication number: WO 2001/038550

(56) References cited:
- OSCAR ZARAGOZA ET AL.: "Disruption of the Candida albicans TPS1 gene encoding trehalose-6-phosphate synthase impairs formation of hyphae and decreases infectivity" JOURNAL OF BACTERIOLOGY, vol. 180, no. 15, August 1998 (1998-08), pages 3809-3815, XP002162880 cited in the application
- SHARKEY L L ET AL: "HWP1 FUNCTIONS IN THE MORPHOLOGICAL DEVELOPMENT OF CANDIDA ALBICANSDOWNSTREAM OF EFG1, TUP1, AND RBF1" JOURNAL OF BACTERIOLOGY,US,WASHINGTON, DC, vol. 1, no. 181, 1999, pages 5273-5279, XP000886181 ISSN: 0021-9193
- LEBERER E ET AL: "SIGNAL TRANSDUCTION THROUGH HOMOLOGS OF THE STE20P AND STE7P PROTEIN KINASES CAN TRIGGER HYPHAL FORMATION IN THE PATHOGENIC FUNGUS CANDIDA ALBICANS" PROCEEDINGS OF THE NATIONAL ACADEMY OF SCIENCES OF USA,US,NATIONAL ACADEMY OF SCIENCE. WASHINGTON, vol. 93, 1 November 1996 (1996-11-01), pages 13217-13222, XP002057299 ISSN: 0027-8424
- EUNICE H. FROELIGER ET L.: "NUT1, a major nitrogen regulatory gene in Magnaporthe grisea, is dispensable for pathogenicity" MOLECULAR AND GENERAL GENETICS, vol. 251, no. 6, 26 July 1996 (1996-07-26), pages 647-656, XP002162881 BERLIN DE
- N.A.R. GOW ET AL.: "Genes associated with dimorphism and virulence of Candida albicans" CANADIAN JOURNAL OF BOTANY, vol. 73, no. 1, 1995, pages S335-S342, XP000990200
- Y.H. FU ET AL.: "Site-directed mutagenesis of the 'zinc finger' DNA-binding domain of he nitrogen-regulatory protein NIT2 of Neurospora" MOLECULAR MICROBIOLOGY, vol. 4, no. 11, 1990, pages 1847-1852, XP000990312
- JANET F. STAAB ET AL.: "Adhesive and mammalian transglutaminase substrate properties of Candida albicans Hwp1" SCIENCE, vol. 283, no. 5407, 5 March 1999 (1999-03-05), pages 1535-1538, XP000887139 DC cited in the application
- STAAB J F ET AL: "YEAST SEQUENCING REPORTS GENETIC ORGANIZATION AND SEQUENCE ANALYSISOF THE HYPHA-SPECIFIC CELL WALL PROTEIN GENE HWP1 OF CANDIDA ALBICANS" YEAST,GB,CHICHESTER, SUSSEX, vol. 14, no. 7, 1998, pages 681-686, XP000886002 ISSN: 0749-503X cited in the application & DATABASE EMBL [Online] Entry CAU64206, 3 April 1997 (1997-04-03) STAAB J.F. ET AL.: "Candida albicans hyphal wall protein 1 (HWP1) gene"
- Euroconference on Fungal Virulence Factors and Disease, Seefeld, Austria, 8-13 September 2001; page 21 LAURA L. SHARKEY ET AL.: "Deceptive correlation of genotype and phenotype at the HWP1 locus"
- Candida and Candidiasis Conference, Tampa, Florida; 13-17 January 2002, page 17 LAURA L. SHARKEY ET AL.:"Deceptive correlation of genotype and phenotype at the HWP1 locus"
- STAAB J.F. ET AL: 'Genetic organization and sequence analysis of the hypha-specific cell wall protein gene HWP1 of Candida albicans' YEAST vol. 14, 1998, pages 681 - 686, XP000886002

## Description

### FIELD OF THE INVENTION

The present invention relates to methodologies and molecular targets for the prevention and treatment of microbial infection of a mammalian host by altering the transcription of hyphal-specific genes. Preferably, these methods and molecular targets may be used in the prevention or treatment of microbial infection of mammalian hosts, such as in immunocompromised patients at risk for opportunistic fungal infections, organ transplant patients, cancer patients undergoing chemotherapy, burn patients, AIDS patients, or patients with diabetic ketoacidosis.

### BACKGROUND OF THE INVENTION

Fungi are not only important human and animal pathogens, but they are also among the most common causes of plant disease. Fungal infections (mycoses) are becoming a major concern for a number of reasons, including the limited number of antifungal agents available, the increasing incidence of species resistant to known antifungal agents, and the growing population of immunocompromised patients at risk for opportunistic fungal infections, such as organ transplant patients, cancer patients undergoing chemotherapy, burn patients, AIDS patients, or patients with diabetic ketoacidosis. The most common clinical isolate is *Candida albicans* (comprising about 19% of all isolates). In one study, nearly 40% of all deaths from hospital-acquired infections were due to fungi. Sternberg, 266(5191) SCIENCE 1632-34 (1994).

The yeast *Candida albicans* (*C. albicans*) is one of the most pervasive fungal pathogens in humans. It has the capacity to opportunistically infect a diverse spectrum of compromised hosts, and to invade many diverse tissues in the human body. It can in many instances evade antibiotic treatment and the immune system. Although *C. albicans* is a member of the normal flora of the mucous membranes in the respiratory, gastrointestinal, and female genital tracts, in such locations it may gain dominance and be associated with pathologic conditions. Sometimes it produces progressive systemic disease in debilitated or immunosuppressed patients, particularly if cell-mediated immunity is impaired. Sepsis may occur in patients with compromised cellular immunity, e.g., those undergoing cancer chemotherapy or those with lymphoma, AIDS, or other conditions. Candida may produce bloodstream invasion, thrombophlebitis, endocarditis, or infection of the eyes and virtually any organ or tissue when introduced intravenously, e.g., via tubing, needles, narcotics abuse, etc.

*C*. *albicans* has been shown to be diploid with balanced lethals, and therefore probably does not go through a sexual phase or meiotic cycle. This yeast appears to be able to spontaneously and reversibly switch at high frequency between at least seven general phenotypes. Switching has been shown to occur not only in standard laboratory strains, but also in strains isolated from the mouths of healthy individuals.

Oropharyngeal and esophageal candidiasis are among the most frequent opportunistic fungal infections observed in human immunodeficiency virus positive (HIV+) and AIDS patients, occurring in the majority of patients. The pathogenesis is complex and is thought to involve multiple host factors that include loss of cell mediated immunity and altered phagocytic cell activity. The current status of the AIDS epidemic is one of increasing numbers of individuals infected and no cure. Many infected individuals may live for a long time with HIV in an essentially permanent immunocompromised state. Because of the loss of the cellular component of the immune system, AIDS patients are susceptible to invasion of submucosal tissue by *C*. *albicans.* The frequency of candidal infections may also be a result of the prophylactic use of antibacterial drugs used in AIDS patients to minimize other opportunistic infections. Candidal infections increase in severity and recur more frequently as the immunodeficiency progresses. Although *C. albicans* is sensitive to antifungal drugs, treatment over long periods of time are required, and isolates from HIV infected patients may be more resistant that other isolates.

In addition to HIV affected patients, oral candidiasis occurs in cancer patients, e.g., leukemia patients, as well as in patients with other underlying diseases. The most commonly associated disease with oral candidiasis, however, is denture stomatites, which is generally observed in denture wearers.

A feature of *C. albicans* growth that is correlated with pathogenicity in the oral cavity is the ability to transform from budding to filament-extending growth. Filamentous forms adhere more readily to buccal epithelial cells (BECs) than budding yeasts, and histologically are a prominent feature of invasion of the mucosa. Alternatively, in mucosal and systemic disease, *C. albicans* exists as a polymorphic set of growth forms termed yeasts, pseudohyphae and true hyphae. In mucosal disease, filamentous forms, particularly true hyphae, invade the keratinized layer of differentiated, stratified squamous epithelium. True hyphae are septate, cylindrical structures with parallel sides that are formed by extension of germ tubes which emerge from yeasts in appropriate environmental conditions. Therefore, knowledge of the molecular events that transform *C. albicans* to the pathogenic filamentous form as well as detailed investigations of the hyphal surface at the molecular level are necessary for understanding the pathogenesis of candidiasis.

If left untreated, Candida infections frequently lead to the death of the patients. Nystatin, ketoconazole, and amphotericin B are drugs which are used to treat oral and systemic Candida infections. However, orally administered nystatin is limited to treatment within the gut and is not applicable to systemic treatment. Some systemic infections are susceptible to treatment with ketoconazole or amphotericin B, but these drugs may not be effective in such treatment unless combined with additional drugs. Amphotericin B has a relatively narrow therapeutic index and numerous undesireable side effects and toxicities occur even at therapeutic concentrations. While ketoconazole and other azole antifungals exhibit significantly lower toxicity, their mechanism of action, inactivation of cytochrome P₄₅₀ prosthetic group in certain enzymes (some of which are found in humans) precludes use in patients that are simultaneously receiving other drugs that are metabolized by the body's cytochrome P₄₅₀ enzymes. *See,* e.g., U.S. Pat. No. 5,863,762.

Other known antifungal agents include: polyene derivatives, such as amphotericin B (including lipid or liposomal formulations thereof) and the structurally related compounds nystatin and pimaricin; flucytosine (5-fluorocytosine); azole derivatives (including ketoconazole, clotrimazole, miconazole, econazole, butoconazole, oxiconazole, sulconazole, tioconazole, terconazole, fluconazole, itraconazole, voriconazole [Pfizer] and SCH56592 [Schering-Plough]); allylamines-thiocarbamates (including tolnaftate, naftifine and terbinafine); griseofulvin; ciclopirox; haloprogin; echinocandins (including MK-0991 [Merck]); nikkomycins; and, bactericidal/permeability-increasing protein (BPI), described in U.S. Pat. Nos. 5,627,153; 5,858,974; 5,652,332; 5,763,567; and 5,733,872.

Resistance of bacteria and other pathogenic organisms to antimicrobial agents is an increasingly troublesome problem. The accelerating development of antibiotic-resistant bacteria, intensified by the widespread use of antibiotics in farm animals and overprescription of antibiotics by physicians, has been accompanied by declining research into new antibiotics with different modes of action. Travis J., 264(5157) Science 360-374 (1994).

Indeed, there is a need for an effective treatment of opportunistic infections caused by *C. albicans* for the aforementioned reasons. Therefore, one objective of the present invention is to provide screening assays for identifying potential inhibitors of *C. albicans* growth. Another object of the present invention is to identify potential inhibitors of *C*. *albicans* growth that are based on the alteration of transcription in this organism.

Whether pathogenic or opportunistic, microorganisms have evolved numerous mechanisms to facilitate their establishment and proliferation in mammalian hosts. For example, during initial infection, the interaction between the microorganism and the host may include attachment or adhesion of the microorganism to the host cell surface, invasion of the host cells by the microorganism, and an elaboration of toxins by the microorganism. In certain instances, the microorganism-host cell interaction may be specific or non-specific. Typically, a specific microorganism-host cell interaction may involve the specific binding of the microorganism to a specific receptor or receptor complex expressed on the host cell surface. Subsequently, the binding event may trigger changes in the microorganism and/or the host cell, leading to the progression of infection.

The role of host cell molecules involved in certain microbial interactions has been determined in some cases. Mammalian transglutaminases are examples for which the molecular mechanisms of action and/or the role in host cell growth and development have been elucidated. Generally, transglutaminases are enzymes that catalyze intermolecular crosslinks by the formation of highly stable isopeptide bonds between the γ-carbonyl group of glutamine and the ε-amino group of lysine residues, which are resistance to proteases, sodium dodecyl sulfate, and heat. Epithelial cell transglutaminases are important for the formation of the cornified envelopes of mature squamous epithelial cells.

Investigators have shown that certain microorganisms may express proteins capable of acting as substrates for mammalian transglutaminases. One example is the hypha-specific hyphal wall protein 1 (HWP1) of *C*. *albicans. HWP1* is a developmentally-regulated cell wall protein that is expressed exclusively in true hypha in cultures and in animal tissues. Staab et al., 283(5407) SCIENCE 1535-38 (1999); Staab et al., 271(11) J. BIOL. CHEM. 6298-305 (1996); Staab & Sundstrom, 14(7) YEAST 681-86 (1998).

Shockey et al. 1(181) J. Bacteriol. 5273-79, 1999, discloses the identification of hwp1 as a developmentally - related hypha- specific gene

*HWP1* consists of an N-terminal proline and glutamine-rich repetitive amino acid sequence, which is exposed on the hyphal surface, and a cell wall-anchored serine and threonine rich C-terminus. The composition of the N-terminal amino acid repeat is reminiscent of mammalian transglutaminase substrates. *HWP1* has a profound effect on adherence of *C*. *albicans* to BECs through a novel mechanism that involves host enzymes. Indeed, *HWP1* may serve as a substrate in transglutaminase-mediated cross-linking reactions, thereby forming isopeptide bonds to proteins on the surfaces of host cells. Furthermore, *HWP*1 encodes an uncoventional adhesin that is necessary for the pathogenesis of candidiasis.

Therefore, by defining the molecular events leading to the expression of a pathogenically important adhesin, and through the identification of new genes that are co-regulated with *HWP*1 in a putative global regulatory circuit, the present invention has strong potential for identifying new and novel ways to interfere with candidiasis. The long term medical benefits of the present invention may be the development of alternative or adjunctive therapies based on new knowledge about expression of hypha-specific genes in *C*. *albicans.*

Accordingly, an objective of the present invention includes identifying and characterizing the 5' and 3' sequences flanking the *HWP1* structural gene. An additional objective of the present invention includes the indentification and characterization of DNA binding proteins (DNA BP) that regulate transcription of *HWP*1*.*

Other objects, features and advantages of the present invention will become apparent from the following detailed description. The detailed description and the specific examples, however, indicate only preferred embodiments of the invention.

### SUMMARY OF THE INVENTION

A specific embodiment of the present invention relates to a method as defined in the claims. Also disclosed is a method for interfering with the expression of hyphal-specific genes in a fungus, which results in the inhibition of the growth of the fungus. This particular embodiment of the present invention is accomplished by interfering with the transcription of hyphal-specific genes mediated by *cis* acting sequences. In a particular embodiment, the fungus comprises a pathogenic or nonpathogenic yeast strain. Specifically, the pathogenic fungus comprises *C*. *albicans.* In a further embodiment of the present invention, the *cis* acting sequences comprises *cis*-regulatory sequences, specifically, a UAS and a URS.

In another embodiment of the present invention, the interfering step comprises interfering with DNA BP that bind to the *cis*-regulatory elements. Further, the interfering step may comprise interfering with the regulatory proteins specific to the DNA BP. Further yet, the interfering step may comprise manipulating factors that interfere with the transcription of the hyphal-specific genes. Finally, the interfering step may comprise manipulating the signal transduction pathways that influence the transcription of the hyphal-specific genes.

In an alternative embodiment, the present invention discloses a method of interfering with the DNA BP by manipulating the binding of the DNA BP to the *cis-*regulatory elements. Further, this embodiment may comprise interfering with the expression of the DNA BP or by altering the ability of the DNA BP to bind to the target *cis*-regulatory element. Further yet, the *cis*-regulatory elements comprise the NIT2 binding site.

In an additional embodiment of the present invention, the DNA BP is encoded by a nucleotide sequence for the DNA binding domain that is homologous to a nucleotide sequence that encodes the DNA binding domain of the NIT2 binding proteins. Specifically, the NIT2 binding domain comprises the protein sequence listed in SEQ. ID. NOS: 2 or 3. The DNA BP may be selected from the group consisting of GAT99, GAT1, and GATA-like binding proteins.

In a further embodiment, the present invention discloses manipulating environmental factors. Regulation of the environmental factors may comprise regulating the environmental temperature of the fungus and/or modifying the nitrogen available to the fungus. A further embodiment disclosed in the present invention comprises manipulating the signal transduction pathways that influence the transcription of the hyphal-specific genes. Specifically, the signal transduction pathway comprises the cAMP-dependant signalling pathway.

In a particular embodiment of the present invention, the hyphal-specific genes may be selected from the group consisting of *HYR*1*, ECE*1*, ALS3,* CHS*2*, and SA*P*6. Specifically, the hyphal-specific gene of the present invention comprises *HWP*1. In a further embodiment of the present invention, the 5' and 3' flanking sequences have been determined. Specifically, the 5' flanking sequence of *HWP*1 comprises the sequence listed in SEQ. ID. NO: 1 and the 3' flanking region comprises the sequence listed in SEQ. ID. NO: 4. Further, the hyphal-specific genes comprises genes responsible for controlling dimorphism. In particular the hyphal-specific gene, *HWP*1 encodes an unconventional adhesin that may be essential for adhesion of the fungus to the patient.

In yet a further embodiment of the present invention, the patients may be immunocompromised and at risk for opportunistic fungal infections. In particular the patient may be, but is not limited to, an organ transplant recipient, a cancer patient undergoing chemotherapy, a burn patient, an AIDS patients, or a patient with diabetic ketoacidosis.

In a final embodiment, the present invention provides a methodology for characterizing genes under the control of DNA BP in a fungus. This embodiment is accomplished by creating a genomic library isolated from a fungus, specifically *C. albicans,* screening the genomic library, and isolating and sequencing the resultant clones.

### BRIEF DESCRIPTION OF THE DRAWINGS

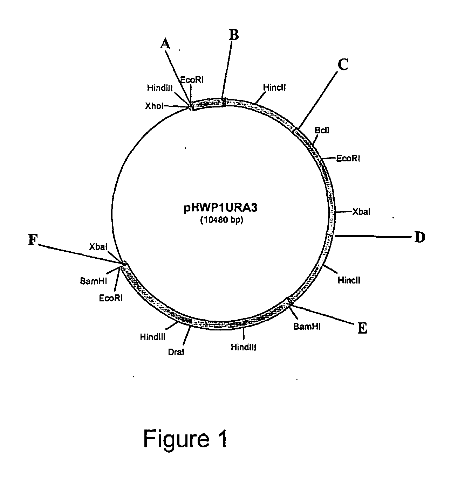

**FIGURE 1** illustrates the pHWP1URA3 plasmid containing an interrupted *HWP1* gene. The plasmid was constructed by first isolating two genomic BamHI fragments containing *HWP*1 half clones from a genomic library constructed in Lambda GEM-12. The BamHI fragments were then cloned in pBluescriptSK⁻ (Stratagene, La Jolla, CA) to generate a 1.0 kb insert and a 3.0 kb insert. An uninterrupted *HWP*1 gene was created by ligating the 3.0 kb insert to create pGBHWP1. An inactive *HWP*1 gene was created by replacing *HWP*1 DNA between the Bcl I and Bgl II sites (394 bp) with the 4.0 kb hisG-URA3-hisG cassette. The segments from A to B and E to F are *HWP*1 DNA; the segment from B to C and D to E are the hisG repeats; and the segment from C to D is URA3. Digestion with Hind III released the disruption cassette from the vector sequences.

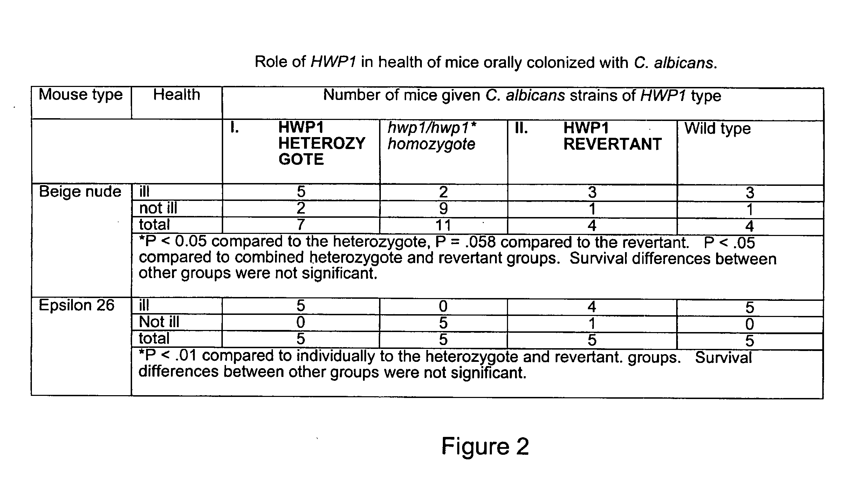

**FIGURE 2** illustrates the role of *HWP1* in health of mice orally colonized with *C. albicans.* Gnotobiotic nude mice that received *C. albicans* strains with *HWP1* were more prone to failure to thrive than mice that received the *C. albicans hwp*1 null mutant strain. Only two of seven beige nude mice given the *HWP1*-containing heterozygous *C. albicans* strain and one of four mice given the *HWP1* revertant remained healthy for six weeks. Only two of 11 mice that received the *hwp1* null mutant strain of *C. albicans* became ill. The survival difference between groups of mice that received the *HWP1* heterozygote strain CAH7 compared to the *hwp1* null mutant was statistically significant ( P < 0.05). The combined groups of mice that received the *HWP1* containing heterozygous and revertant, also showed a significant difference in survival from mice receiving the *hwp1* null mutant strain. The absence of a significant difference in the survival of mice that received the revertant strain and the *hwp1* null mutant (P = 0.058) was because one of the mice was sacrificed for "comparison" prior to becoming ill. When this mouse is removed from the analysis, the survival difference between null and revertant is significant. The survival of Epsilon 26 mice also depended on the presence of *HWP1* in *C. albicans* strains used for monoassociation. All mice that received *HWP1-*containing heterozygous *C. albicans* strains and four of five mice given the *HWP1* revertant strain became ill. Only one mouse that received the revertant strain remained healthy for seven weeks. In contrast, none of the five mice that received the *hwp1* null mutant strains of *C. albicans* became ill. The survival differences between groups of mice that received the *HWP1* heterozygote and revertant strains compared to the *hwp1* null mutant were statistically significant ( P < 0.01).

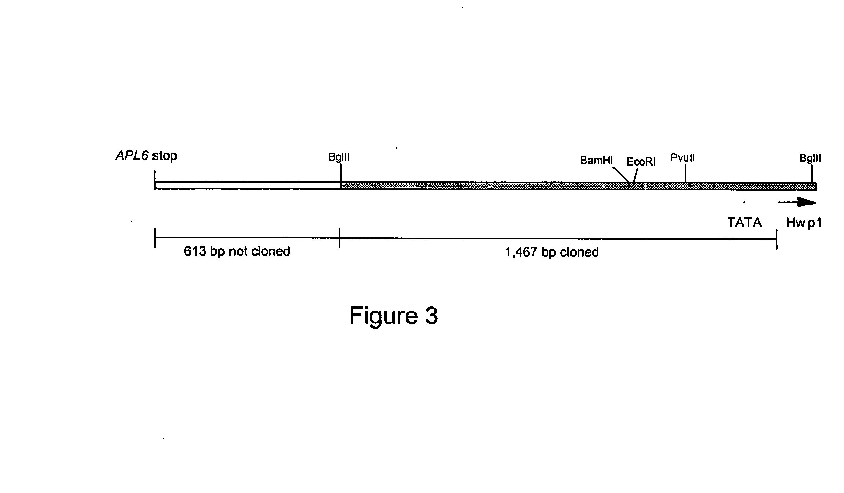

**FIGURE 3** depicts the sequence of the 5' flanking region of *HWP1.* The upstream region of *HWP*1 containing the regulatory sequences has been isolated as a 1.6 Bgl II fragment of *C. albicans* genomic DNA. The cloned *HWP*1 promoter region includes 1.467 kb upstream of the ATG start codon of the structural *HWP1* gene. A putative TATA box was found 67 bp upstream form the beginning of the transcript.

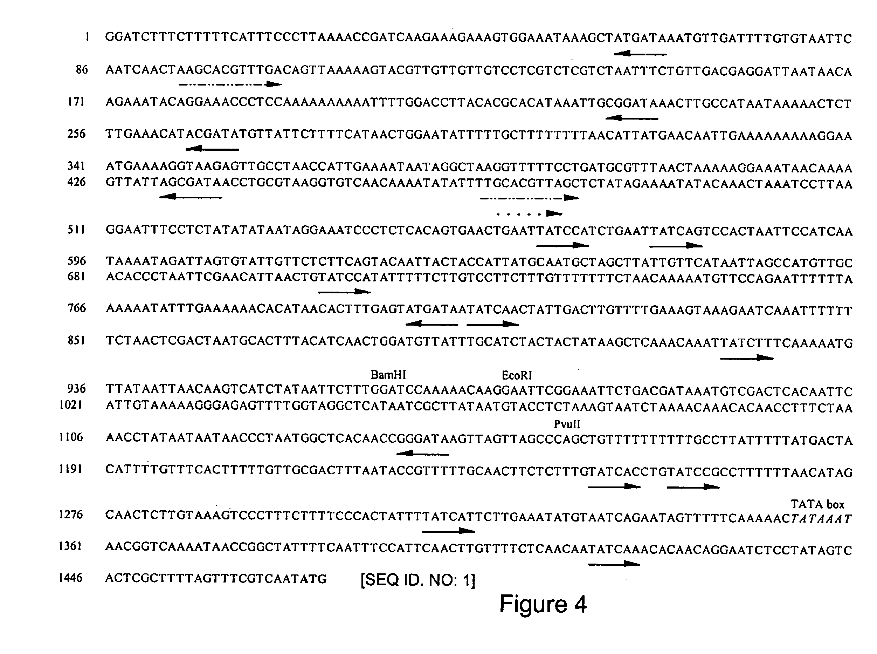

**FIGURE 4** illustrates the features of the clones 5' flanking region of the *HWP1* gene [SEQ. ID. NO: 1]. A 10 nucleotide perfect direct repeat was found at positions 554 and 567. A TATA box was found 67 bp upstream from the beginning of the transcript. A search using the MatInspector v2.2 program revealed the presence of 16 NIT2 sites, 2 PHO4 sites, 1 MATA1 site, and 1 HSF site within the 1,467 bp 5' flanking region. The position and direction of the NIT2 sites are shown with → below the sequence, the PHO4 sites are designated by →, and the CRE site within the PHO4 site is shown by →. A notable feature is a pair of NIT2 sites in opposite orientations near position 800 that form an almost perfect palindrome on opposite DNA strands.

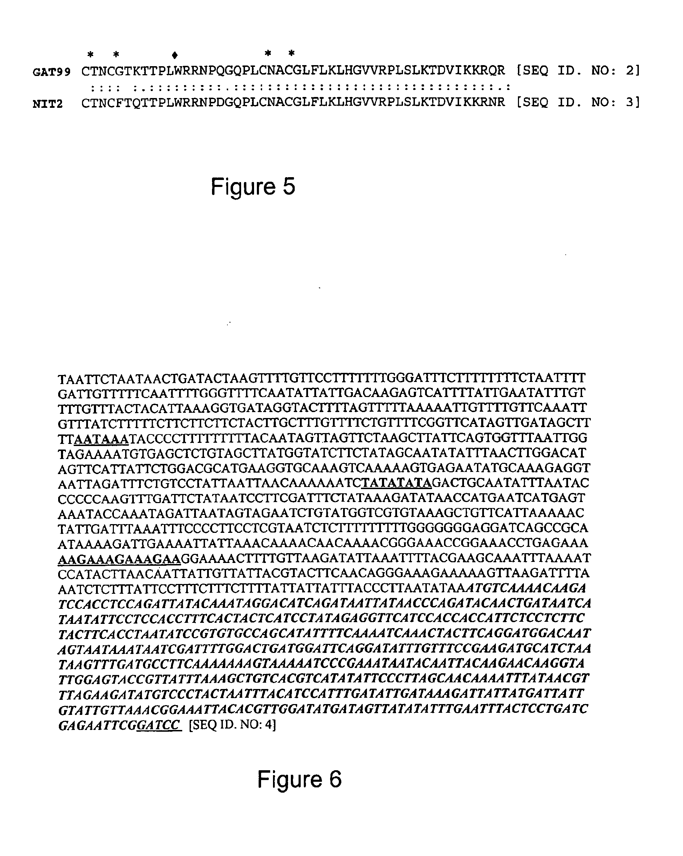

**FIGURE 5** illustrates the comparison between the GAT99 50-residue DNA binding domain [SEQ. ID. NO: 2] with the NIT2 50-residue DNA binding domain [SEQ. ID. NO: 3]. A search of the *C. albicans* genome revealed that GAT99 is highly homologous to NIT2. Using the ALIGN program, 92% homology was observed between the two DNA binding domains. The asterisks (*) indicate cysteine residues involved in zinc chelation, and the diamond (◆) identifies the leucine residue, which when altered in AREA and NIT2 led to different changes in promoter recognition.
**FIGURE 6** illustrates 1,441 nucleotides of the 3'flanking region of *HWP1* [SEQ. ID. NO: 4]. Translation of *HWP1* ends with a UAA codon followed with 277 nucleotides of untranslated message. A polyadenylation site (AAUAAA) 252 nucleotides from the nucleotides from the stop codon, and a poly A tail of adenosines are also part of the 3' region of the message. The polyadenylation site and other sequences potentially important for 3' end formation are shown in bold. A truncated open reading frame (bold-italics) consisting of 178 amino acids, beginning with ATG and interrupted by the BamHI site at the end of the clone (underlined), exists at the 3' end of this region indicating the presence of an additional gene.

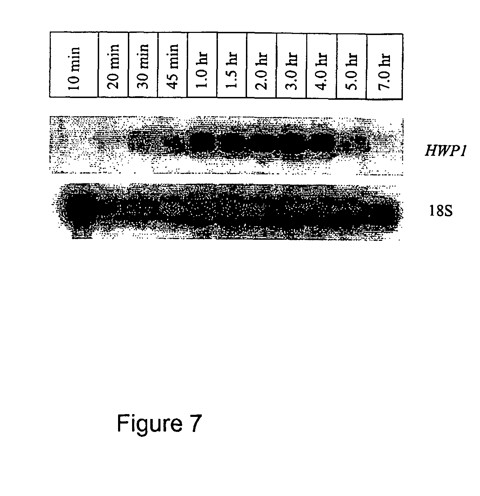

**FIGURE 7** depicts a Northern blot of *HWP1* mRNA three hours after initiation of mass conversion from yeasts to hyphae (strain SC5314) in M199. Each lanes contains 2.5 µg of total RNA. To confirm the presence of equivalent amounts of RNA in each lane, an 18S ribosomal probe was used as a control. The blots were first probed with *HWP1* cDNA which represents he 5' region of the structural gene. The blot has been overexposed to show that *HWP*1 mRNA is detectable within 20 minutes and at 7 hrs of placing cells under induction conditions (*HWP*1 mRNA is clearly visible in the autoradiograph, but is faint in the reproduction). The message level increases dramatically between 20 and 30 minutes and continues to rise, peaking at 3-4 hours. After 4 hours, the *HWP*1 mRNA begins to decline until 7 hours where it stabilizes at a low level.

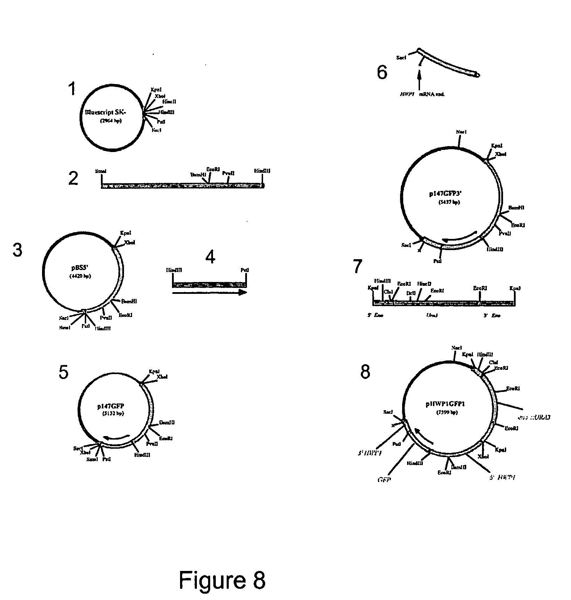

**FIGURE 8** illustrates the plasmid construction for the *HWP1* promoter studies. 1. PBluescriptSK- was used as the backbone. 2. The entire region of *HWP1* was amplified using oligonucleotides that incorporated Sma I and Hind III sites to the ends. The PCR product was then digested with Hind III and Sma I. 3. The digested PCR product was cloned into pBluescript digested with Hinc II and Hind III, creating pBS5'. 4. A GFP gene fragment was produced by digesting pYGFP3 with Hind III and Pst I. 5. The digested GFP fragment was cloned into pBS5' that had been digested with Hind III and Pst I, creating p147GFP. 6. The 3' region of *HWP1* was amplified with an oligonucleotide that eliminates the Hind III site downstream of the mRNA end. The fragment was exposed to Pfu polymerase to generate blunt ends, followed by digestion with Sac I. The *HWP*1 3' digested fragment was cloned into p147GFP that had been digested with Sma I and Sac I, creating p147GFP3'. This cloning strategy ensured that the *HWP*1 3' end would be inserted into the plasmid in the proper orientation. 7. Eno:URA3 was amplified with oligonucleotides that added Kpn I sites at the ends. The URA3 PCR product was digested with Kpn I and cloned into a Kpn I digested p147GFP3', creating pHWP1GFP1. 8. The final construct, pHPW1GFP1, contains 1,467 bp of *HWP*1 upstream flanking sequences, 352 bp of downstream flanking sequence, and the *C. albicans* enolase gene disrupted with the *C. albicans* URA3 gene. The URA3 gene was used for selection in the *ura3* strain CAI4 and to target one of the ENO gene loci of *C. albicans.*

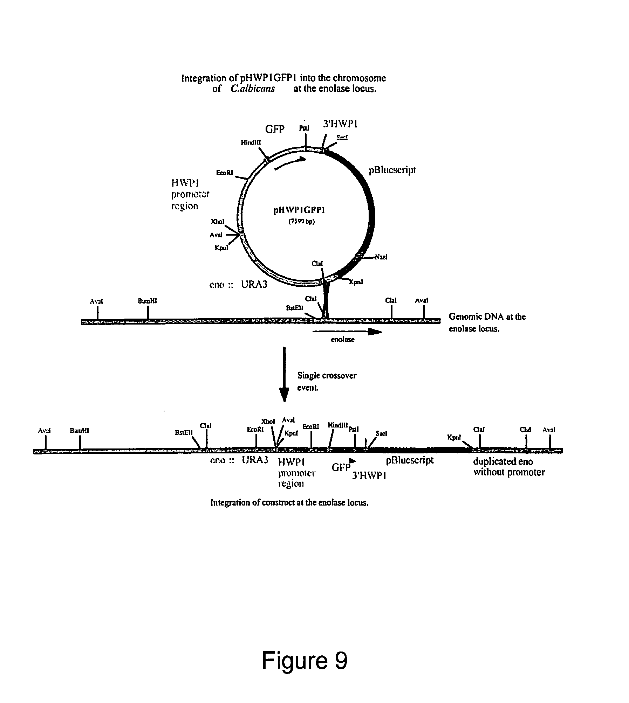

**FIGURE 9** illustrates the integration of pHWP1GFP1 into the chromosome of *C. albicans* at the enolase locus. pHWP1GFP1 was linearized with ClaI and then used to transform the Ura strain CAI4. The linearized plasmid was targeted to the ENO locus in the genome using spheroplast transformation, resulting in a mutated copy of the enolase gene disrupted with URA3 adjacent to a wild type copy. The transformants will be selected on media lacking uridine. A control plasmid lacking *HWP1* upstream region was used as a negative control.

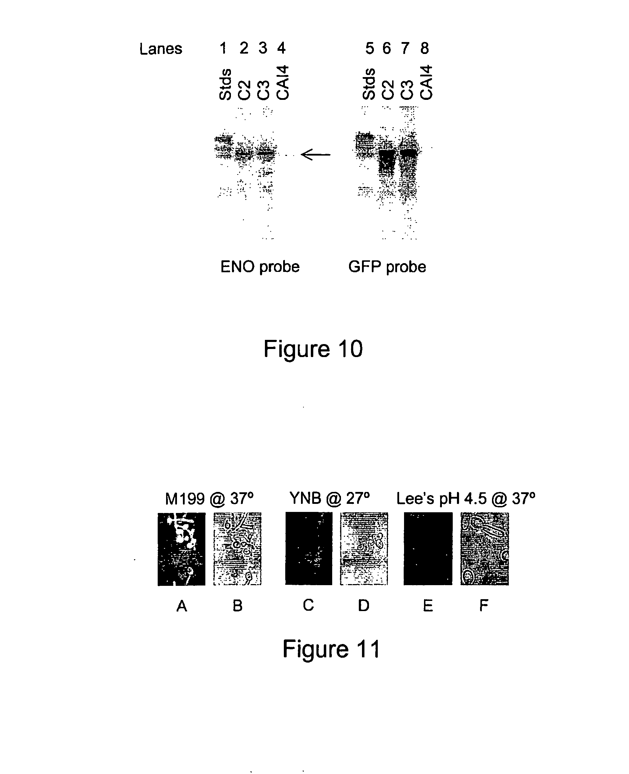

**FIGURE 10** depicts a Southern blot to show the stable integration of the *HWP*1 promoter:GFP construct at the genomic enolase locus in Ura strain CAI4. Following spheroplast transformation, two transformants were produced, GFP1/C2 and GFP1/C3. Southern blotting of genomic DNA digested with Ava I confirmed that transformants C2 and C3 had integrated GFP into the ENO locus of *C*. *albicans.* The integrated construct produced a 7.8 kb Ava I band that was recognized by both the enolase (lanes 2 and 3) and GFP (lanes 6 and 7) probes. The undisrupted enolase locus produced a very faint 6.4 kb band (lane 4, arrow). The GFP band was not detected in the parental strain CAI4, lane 8.
**FIGURE 11** depicts fluorescence microscopy of the GFP-producing transformants. Fluorescence was used to demonstrate that the *HWP*1 promoter:GFP construct is developmentally regulated. The C2 and C3 transformants were grown to stationary phase in YNB for 48 hours at room temperature before placement in M199, fresh YNB at 27°, or in the four Lee's media conditions (pH 4.5 and 6.8 at 27° and 37°). The cells were examined for the production of GFP in germ tubes incubated in M199 and Lee's pH 6.8 at 37°. (A) fluorescence was not localized to the germ tubes but was found throughout the cytoplasm of *C. albicans.* (B) the appearance of fluorescence coincided with the induction of germ tube formation. (C) yeast did not express GFP. (E) pseudohyphae did not express GFP. Panels B, D, and F are controls.

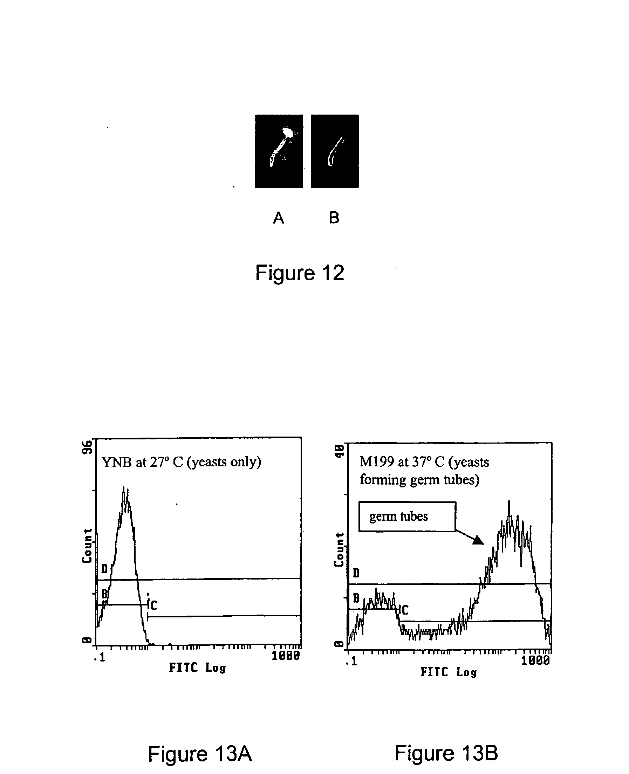

**FIGURE 12** illustrates indirect immunofluorescence (IIF). IIF was used to demonstrate that GFP does not interfere with *HWP*1 expression using GFP-producing transformants. Secondary anti-rabbit antibodies conjugated with Texas Red rather were used so that *Hwp*1 could be detected in the presence of GFP. Panel A shows the GFP-producing transformants. Panel B shows *Hwp*1 of the GFP-producing transformants stained with Texas red.
**FIGURE 13** illustrates flow cytometry to quantify the production of GFP within the C2 and C3 stable tranasformants. The graph in panel B illustrates a three log shift in fluorescence of germ tube compared with the negative control in panel A. Yeast cells that did not germinate were used as the negative control in panel A and as illustrated are non-fluorescent.

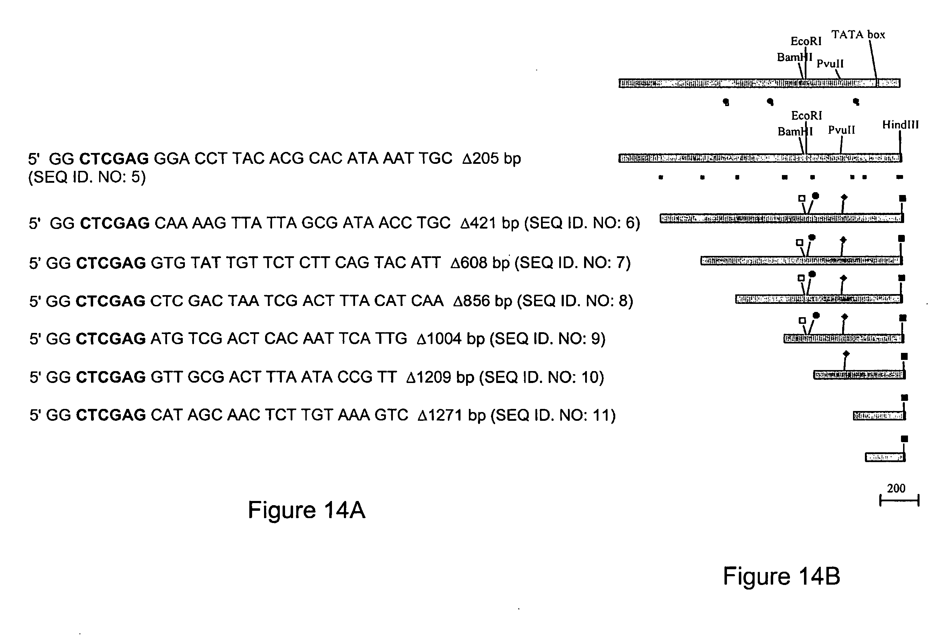

**FIGURE 14** illustrates the nested set of deletions created to identify the location of *cis*-acting regulatory sequences upstream of *HWP*1*.* The sequential deletions of DNA were performed by PCR with oligonucleotides to sequences progressively closer to the -1 site. The same 3' oligonucleotides (shown as black vertical lines I at fragement ends) used to generate the original plamsid construct (pHWP1GFP1) were used. The oligonucleotides for generating the nested deletions are shown in panel A [SEQ. ID. NOS: 5-11]. The bolded sequence is the Xho I restriction enzyme site. Panel B shows the expected PCR products using the oligonucleotides to generate the sequential deletions of 5' *HWP*1*.* The top linear map shows the location of the NIT2 pairs below the sequence as •. The location of the TATA box is also indicated. The effect of the NIT2 binding sites will be detected in Δ608, Δ856, and Δ1271. The second map shows the oligonucleotide binding sites as ■. The smallest PCR product will have 83 bp ofDNA upstream from the TAT box. This construct will serve as a minimal promoter reporter for testing activating functions of regulatory elements.

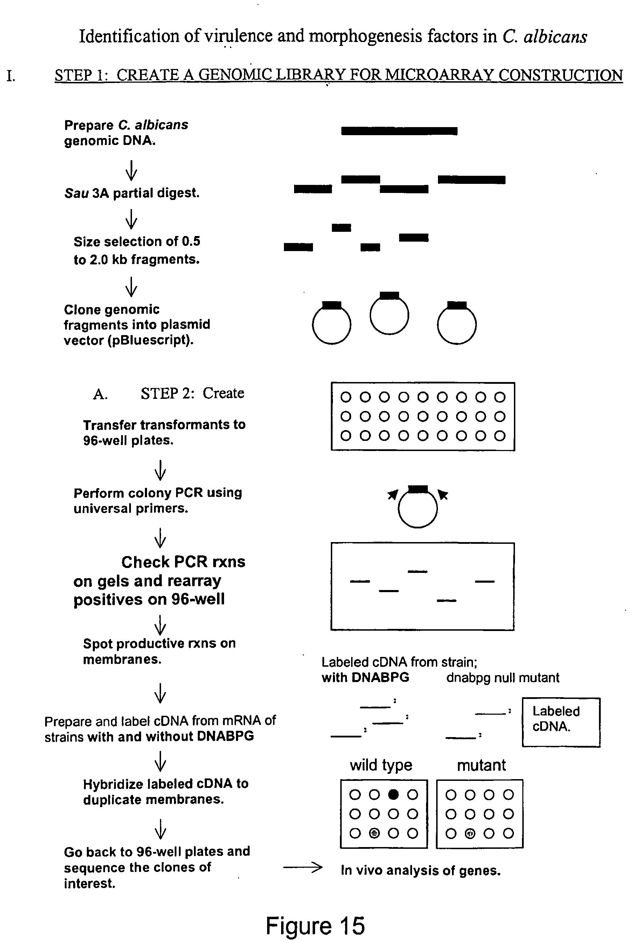

**FIGURE 15** illustrates the mini-array-based, transcription analysis of *C*. *albicans* genomic library to identify and characterize genes under control of DNA binding proteins. This method is more sensitive than differential screening.

### DETAILED DESCRIPTION OF THE INVENTION

It is understood that the present invention is not limited to the particular methodology, protocols, cell lines, vectors, and reagents, etc., described herein, as these may vary. It is also to be understood that the terminology used herein is used for the purpose of describing particular embodiments only, and is not intended to limit the scope of the present invention. It must be noted that as used herein and in the appended claims, the singular forms "a," "an," and "the" include plural reference unless the context clearly dictates otherwise. Thus, for example, a reference to "a DNA BP" is a reference to one or more DNA BPs and equivalents thereof known to those skilled in the art and so forth.

Unless defined otherwise, all technical and scientific terms used herein have the same meanings as commonly understood by one of ordinary skill in the art to which this invention belongs. Preferred methods, devices, and materials are described, although any methods and materials similar or equivalent to those described herein can be used in the practice or testing of the present invention.

### Definitions

**Promoter**: a recognition site on a DNA sequence or group of DNA sequences that provide an expression control element for a gene and to which RNA polymerase specifically binds and initiates RNA synthesis (transcription) of that gene.

**Inducible promoter:** a promoter where the rate of RNA polymerase binding and initiation is modulated by external stimuli. Such stimuli include light, heat, anaerobic stress, alteration in nutrient conditions, presence or absence of a metabolite, presence of a ligand, microbial attack, wounding and the like.

**Viral promoter:** a promoter with a DNA sequence substantially similar to the promoter found at the 5' end of a viral gene. A typical viral promoter is found at the 5' end of the gene coding for the p2I protein of MMTV described by Huang et al., 27(2 Pt 1) CELL 245-55 (1981).

**Synthetic promoter:** a promoter that was chemically synthesized rather than biologically derived. Usually synthetic promoters incorporate sequence changes that optimize the efficiency of RNA polymerase initiation.

**Constitutive promoter:** a promoter where the rate of RNA polymerase binding and initiation is approximately constant and relatively independent of external stimuli.

**Heterologous Polypeptide:** a linear series of amino acid residues connected one to the other by peptide bonds between the alpha-amino and carboxy groups of adjacent residues originating from a species other than the plant host system within which said linear series is produced. "Polypeptide" also encompasses a sequence of amino acids, peptides, fragments of polypeptides, proteins, globular proteins, glycoproteins, and fragments of these.

**Multimeric protein:** a protein containing more than one separate polypeptide or protein chain, each associated with the other to form a single protein. Both heterodimeric and homodimeric proteins are multimeric proteins.

**Immunoglobulin:** a polypeptide, protein or multimeric protein containing at least the immunologically active portion of an immunoglobulin heavy chain and is thus capable of specifically combining with an antigen. Exemplary immunoglobulins are immunoglobulin heavy chains, immunoglobulin molecules, substantially intact immunoglobulin molecules, any portion of an immunoglobulin that contains the paratope, including those portions known in the art as Fab fragments, Fab' fragment, F(ab').sub.2 fragment and Fv fragment.

**Recombinant:** as used herein, broadly describes various technologies whereby genes can be cloned, DNA can be sequenced, and protein products can be produced. As used herein, the term also describes proteins that have been produced following the transfer of genes into the cells of host systems.

**Fusion protein:** a protein in which peptide sequences from different proteins are covalently linked together.

**Hybridization:** broadly defined, any process by which a nucleic acid sequence binds to a complementary sequence through base pairing. Hybridization conditions can be defined by, for example, the concentrations of salt or formamide in the prehybridization and hybridization solutions, or by the hybridization temperature, and are well known in the art. Hybridization can occur under conditions of various stringency. In particular, stringency can be increased by reducing the concentration of salt, increasing the concentration of formamide, or raising the hybridization temperature. For example, hybridization under high stringency conditions could occur in about 50% formamide at about 37°C to 42°C. Hybridization could occur under reduced stringency conditions in about 35% to 25% formamide at about 30°C to 35°C. In particular, hybridization could occur under high stringency conditions at 42°C in 50% formamide, 5 times SSPE, 0.3% SDS, and 200 ug/ml sheared and denatured salmon sperm DNA. Hybridization could occur under reduced stringency conditions as described above, but in 35% formamide at a reduced temperature of 35°C. The temperature range corresponding to a particular level of stringency can be further narrowed by calculating the purine to pyrimidine ratio of the nucleic acid of interest and adjusting the temperature accordingly. To remove nonspecific signals, blots can be sequentially washed, for example, at room temperature under increasingly stringent conditions of up to 0. 1 X saline sodium citrate and 0.5% sodium dodecyl sulfate. Variations on the above ranges and conditions are well known in the art.

**Isolated:** as used herein, refers to any element or compound separated not only from other elements or compounds that are present in the natural source of the element or compound, but also from other elements or compounds and, as used herein, preferably refers to an element or compound found in the presence of (if anything) only a solvent, buffer, ion, or other component normally present in a solution of the same.

**Nucleic acid sequences:** as the term is used herein, nucleic acid sequences encoding a hyphal-specific gene or functional equivalent thereof including those with deletions, insertions, or substitutions of different nucleotides resulting in a polynucleotide that encodes the same or a functionally equivalent or hyphal-specific protein. Included within this definition are polymorphisms which may or may not be readily detectable using a particular oligonucleotide probe of the polynucleotide encoding a protein and improper or unexpected hybridization to alleles, with a locus other than the normal chromosomal locus for the polynucleotide sequence encoding a heterologous polypeptide. The encoded protein may also be "altered" and contain deletions, insertions, or substitutions of amino acid residues which produce a silent change and result in a functionally equivalent protein. Deliberate amino acid substitutions may be made on the basis of similarity in polarity, charge, solubility, hydrophobicity, hydrophilicity, and/or the amphipathic nature of the residues as long as the biological or immunological activity of a protein is retained. For example, negatively charged amino acids may include aspartic acid and glutamic acid; positively charged amino acids may include lysine and arginine; and amino acids with uncharged polar head groups having similar hydrophilicity values may include leucine, isoleucine, and valine, glycine and alanine, asparagine and glutamine, serine and threonine, and phenylalanine and tyrosine.

The term "nucleic acid sequence," includes an oligonucleotide, nucleotide, or polynucleotide, and fragments thereof, and to DNA or RNA of genomic or synthetic origin which may be single- or double-stranded, and represent the sense or antisense strand, to peptide nucleic acid (PNA), or to any DNA-like or RNA-like material, natural or synthetic in origin. "Fragments" include nucleic acid sequences which are greater than about 60 nucleotides than in length, and most preferably includes fragments that are at least about 100 nucleotides, at least about 1000 nucleotides, and at least about 10,000 nucleotides in length.

**Antisense gene:** an antisense gene is constructed by reversing the orientation of the gene with respect to its promoter so that the antisense strand is transcribed.

**Antisense RNA:** an RNA molecule complementary to a particular RNA transcript that can hybridize to the transcript and block its function.

**Amino acid sequences:** as used herein, this term includes an oligopeptide, peptide, polypeptide, or protein sequence, and fragment thereof, and to naturally occurring or synthetic molecules.

**Fragments:** include any portion of an amino acid sequence which retains at least one structural or functional characteristic of the subject heterologous polypeptides.

**Derivative:** as used herein, includes the chemical modification of a nucleic acid encoding or complementary to a hyphal-specific protein polypeptides of the present invention. Such modifications include, for example, replacement of hydrogen by an alkyl, acyl, or amino group. A nucleic acid derivative encodes a polypeptide which retains the biological or immunological function of the natural molecule. A derivative polypeptide is one which is modified by glycosylation, pegylation, or any similar process which retains the biological or immunological function of the polypeptide from which it was derived.

**Chemical derivative:** as used herein, a molecule is said to be a "chemical derivative" of another molecule when it contains additional chemical moieties not normally a part of the molecule. Such moieties can improve the molecule's solubility, absorption, biological half-life, and the like. The moieties can alternatively decrease the toxicity of the molecule, eliminate or attenuate any undesirable side effect of the molecule, and the like.

**Complementary or complementarity:** as used herein, include the natural binding of polynucleotides under permissive salt and temperature conditions by base-pairing. For example, the sequence "A-G-T" binds to the complementary sequence "T-C-A." Complementarity between two single-stranded molecules may be "partial," in which only some of the nucleic acids bind, or it may be complete when total complementarity exists between the single stranded molecules. The degree of complementarity between nucleic acid strands has significant effects on the efficiency and strength of hybridization between nucleic acid strands. This is of particular importance in amplification reactions, which depend upon binding between nucleic acids strands and in the design and use of molecules.

**Deletion:** as used herein, refers to a change in the amino acid or nucleotide sequence and results in the absence of one or more amino acid residues or nucleotides.

**Insertion or addition:** as used herein, includes a change in an amino acid or nucleotide sequence resulting in the addition of one or more amino acid residues or nucleotides, respectively, as compared to the naturally occurring molecule.

**Introduction:** insertion of a nucleic acid sequence into a cell, by methods including infection, transfection, transformation or transduction.

**Transfection:** as used herein includes the process of introducing a DNA expression vector into a cell. Various methods of transfection are possible including microinjection or lipofection.

**Transformation:** a process by which exogenous DNA enters and changes a recipient cell. It may occur under natural or artificial conditions using various methods well known in the art. Transformation may rely on any known method for the insertion of foreign nucleic acid sequences into a prokaryotic or eukaryotic host cell. The method is selected based on the type of host cell being transformed and may include, but is not limited to, viral infection, electroporation, heat shock, and lipofection.

**Functional equivalent:** a protein or nucleic acid molecule that possesses functional or structural characteristics that are substantially similar to a heterologous protein, polypeptide, enzyme, or nucleic acid. A functional equivalent of a protein may contain modifications depending on the necessity of such modifications for the performance of a specific function. The term "functional equivalent" is intended to include the "fragments," "mutants," "hybrids," "variants," "analogs," or "chemical derivatives" of a molecule.

**Variant:** an amino acid sequence that is altered by one or more amino acids. The variant may have "conservative" changes, wherein a substituted amino acid has similar structural or chemical properties, e.g., replacement of leucine with isoleucine. More rarely, a variant may have "nonconservative" changes, e.g., replacement of a glycine with a tryptophan. Analogous minor variations may also include amino acid deletions or insertions, or both. Guidance in determining which amino acid residues may be substituted, inserted, or deleted may be found using computer programs well known in the art, for example, DNASTAR® software.

**% similarity or % identity:** refer to the percentage of sequence similarity or identity found in a comparison of two or more amino acid or nucleic acid sequences. Percent similarity can be determined by methods well-known in the art. For example, percent similarity between amino acid sequences can be calculated using the clustal method. *See, e.g.,* Higgins & Sharp, 73 GENE 237-44 (1988). The clustal algorithm groups sequences into clusters by examining the distances between all pairs. The clusters are aligned pairwise and then in groups. The percentage similarity between two amino acid sequences, e.g., sequence A and sequence B, is calculated by dividing the length of sequence A, minus the number of gap residues in sequence A, minus the number of gap residues in sequence B, into the sum of the residue matches between sequence A and sequence B, times one hundred. Gaps of low or of no homology between the two amino acid sequences are not included in determining percentage similarity. Percent similarity can be calculated by other methods known in the art, for example, by varying hybridization conditions, and can be calculated electronically using programs such as the MEGALIGN^{™} program. (DNASTAR Inc., Madison. Wisconsin.)

**Operably linked:** as used herein, refers to the state of any compound, including but not limited to deoxyribonucleic acid, when such compound is functionally linked to any promoter. In the context of the present invention, the nucleic acid sequence is one that encodes for a hyphal-specific protein. The promoter sequence initiates and mediates transcription of the nucleic acid sequence.

**Vector:** a cloning vector that is designed so that a coding nucleic acid sequence inserted at a particular site will be transcribed and translated. A typical expression vector may contain a promoter, selection marker, nucleic acids encoding signal sequences, and regulatory sequences, e.g., polyadenylation sites, 5'-untranslated regions, and 3'-untranslated regions, termination sites, and enhancers. "Vectors" include viral derived vectors, bacterial derived vectors, plant derived vectors and insect derived vectors.

**Protein purification**: broadly defined, any process by which proteins are separated from other elements or compounds on the basis of charge, molecular size, or binding affinity.

**Substantially purified:** as used herein, includes nucleic or amino acid sequences that are removed from their natural environment, isolated or separated, and are at least 60% free, preferably at least 75% free, and most preferably at least 90% free from other components with which they are naturally associated.

**Expression cassette:** is conventional and refers to a combination of regulatory elements that are required by the host for the correct transcription and translation (expression) of the genetic information contained in the expression cassette. These regulatory elements comprise a suitable (i.e., functional in the selected host) transcription promoter and a suitable transcription termination sequence.

**Promoter:** generally includes a regulatory region of DNA capable of initiating, directing and mediating the transcription of a nucleic acid sequence. Promoters may additionally comprise recognition sequences, such as upstream or downstream promoter elements, which may influence the transcription rate. Preferably, in the context of the present invention, the promoters are hyphal-specific gene promoters.

**Inhibition:** as used herein, refers to a reduction in the parameter being measured, whether it be *C. albicans* growth or viability. The amount of such reduction is measured relative to a standard (control). The prefered detection products may include newly transcribed mRNA and a DNA-DNABP complex. "Reduction" is defined herein as a decrease of at least around 25% relative to control, preferably at least around 50%, and most preferably of at least aound 75%.

**DNA template:** refers to double-stranded DNA and where indicated by the particular binding assay to single-stranded DNA that may be negatively supercoiled, possesses a promoter region.

**Formation of a complex:** refers to the binding of a transcription factor(s) to a DNA template.

**Cis-acting element:** refers to a variety of modular elements or target sequences. These elements may be targets for tissue-specific or temporal regulation. Generally, these elements only affect the activity of DNA sequences of its own DNA molecule. These elements may be found within enhancers, promoters, or other regulatory elements of a particular gene.

**DNA binding protein:** refers to transcription factors and other regulatory proteins that recognize specific target sequences located in enhancers, promoters, or other regulatory elements that affect a particular target gene. For a repressor protein, binding to DNA may itself be sufficient to excersise its function, e.g., inhibit transcription. Alternatively, to be a positive regulatory protein, the DNABP may excerise its function by binding to regulatory proteins or other transcription factors.

In accordance with the present invention, a patient preferably includes immunocompromised or immunosuppressed humans, for example, those having AIDS or undergoing transplantation or anti-cancer therapy. The invention also preferably relates to humans with primary or secondary immunodeficiencies (MERCK MANUAL 16th ed., Chapter 19 (1992)). In addition to mammalian hosts in which the normal immune response has been compromised or suppressed, the invention relates to mammalian hosts in which the normal microbial flora has been disrupted, for example, because of disease (e.g., hereditary, metabolic, infiltrative, or hematologic), trauma (e.g., burn, splenectomy, anesthesia), surgical or clinical procedure (e.g., catheterization or introduction of artificial implants such as dentures), or chemical, radiation, or other immunosuppressive prophylaxis or treatment. Accordingly, the microbial infection of the present invention includes infections related to opportunistic as well as pathogenic microorganisms.

A prefered embodiment of the present invention involves defining the molecular events leading to the expression of a pathogenically important adhesin. In a particular embodiment of the present invention, this will be accomplished by indentifying new genes that are co-regulated with *HWP*1 in a putative global regulatory circuit. In a further embodiment, the present invention proposes novel means of interfering with the regulation of transcription of these new genes. Preferably, interfering with the regulation of transcription of hyphal-specific genes, specifically *HWP*1*.̅* Finally, another emodiment of the present invention may be used in the development of an alternative or adjunctive therapy for candidiasis infections.

A hallmark of tissue invasion by the yeast *C. albicans* is the preponderance of hyphal forms during growth within tissue. This observation prompts the hypothesis that the ability to form hyphae is responsible for the pathogenesis of candidiasis. However, several lines of evidence suggest that it is not necessarily growth as hyphae that is essential for disease, but rather it is the expression of pro-invasive factors that coincide with hyphal growth that mediates tissue invasion. In some cases, the lack of filament production is correlated with a loss in virulence (Csank et al., 66(6) INFECT. IMMUN. 2713-21 (1998); Ghannoum et al., 63(11) INFECT. IMMUN. 4528-30 (1995); Lo et al., 90(5) CELL 939-49 (1997); Zaragoza et al., 180(15) J. BACTERIOL. 3809-15 (1998)) but other examples exist where deletion of a specific gene product does not impair hyphal growth in tissues or in laboratory cultures, yet virulence is reduced in animal models. De Bernardis et al. 179(1) J. INFECT. DIS. 201-8 (1999); Leidich et al., 273(40) J. BIOL. CHEM. 26078-86 (1998). Moreover, a mutant that grows obligately in filamentous forms does not show enhanced virulence. Braun & Johnson, 277(5322) SCIENCE 105-09 (1997); De Bernardis et al.; Leidich et al. Although some studies have suggested that mutant strains lacking the ability to form hyphae are avirulent, it is not known whether the loss of virulence is a result of the lack or hyphae *per se,* or a lack of specific pro-invasive factors that are expressed during hyphal growth. Finally, studies have shown that strains of *C. albicans* isolated from human patients that are uniformly succesful in forming hyphae, vary in their ability to invade tissue in a rat tongue model. Allen et al., 18(6) J. ORAL PATHOL. MED. 352-59 (1990). These observations all point to the presence of pro-invasive factors that if present in a strain, are produced during hyphal growth. Thus hyphal growth may be necessary but not sufficient for candidiasis to occur. In order to understand the pathogenesis of candidiasis, it is important to identify proteins that contribute to pathogenesis, and then to discover factors that regulate their expression. The ability to interfere with mechanisms of expression of pro-invasive genes will lead to the development of strategies to interfere with candidiasis.

*HWP*1 is a developmentally-regulated cell wall protein that is expressed exclusively in true hyphae in cultures and in animal tissues. Hwp1 has a profound effect on adherence to human BECs through a novel mechanism that involves host enzymes, and is important for systemic candidiasis in mice. *HWP1* expression is tightly regulated at the level of mRNA. Experiments using cultures growing exclusively as yeasts lack *HWP1* mRNA, whereas in cultures of true hyphae, *HWP*1 mRNA is abundant, *e.g,.* even more abundant than genes for glycolytic enzymes. The most plausible explanation for developmental expression is differences in transcription of *HWP1* in yeast and hyphal growth forms. The hypothesis is that flanking regions of the *HWP1* structural gene contain regulatory sequences that affect the levels of *HWP1* mRNA. A corollary is that *HWP1* is a target of a gene regulatory circuit that also controls expression of other hypha-specific genes that are pathogenically important.

Hypha-specific genes, i.e., genes that are expressed in hyphal growth, but not in yeast growth, are important virulence factors. Bailey et al., 178(18) J. BACTERIOL. 5353-60 (1996); Birse et al., 61(9) INFECT. IMMUN. 3648-55 (1993); Staab et al. (1996). The hypha-specific gene *HWP1* encodes an unconventional adhesin that is necessary for the pathogenesis of candidiasis. Staab et al. (1999). HWP1 attaches tightly to host cells by virtue of being a substrate for mammalian transglutaminase, thereby forming isodipeptide bonds to proteins on surfaces of host cells. Besides being an adhesin, HWP1 influences germ tube formation in the special case where blastospores are dropped on the surface of an agar plate. Sharkley et al. 181(17) J. BACTERIOL. 5273-9 (1999). It is possible that the defective germ tube formation on surfaces of agar of the null mutant may be indicative of a defect in germ tube formation in growth in host tissues; but if so, the defect is subtle because germ tube formation in animal tissues, in liquid cultures, and in embedded agar media are normal for the *hwp1* null mutant. It is more likely that the null mutant is avirulent because of defects in adherence to host cells. Other hypha-specific genes include an aspartyl proteinase (SAP6) and proteins predicted to be localized to the cell wall (ALS3 and HYR). Bailey et al.; Hoyer et al., 33(6) CURR. GENET. 451-59 (1998). Proteinase activity and conventional adherence through cell wall proteins other than HWP1 are potentially important virulence attributes. Given the importance of HWP1 in pathogenesis, and the potential roles for other hypha-specific proteins in pathogenesis based on their probable functions, the mechanisms regulating hypha-specific gene expression are of interest for defining strategies to inhibit candidiasis.

The regulation of expression of hypha-specific genes is controlled by the presence of mRNA in hyphae but not in yeasts. The presence of multiple genes that are correlated with hyphal growth, and the common control determined by the presence of mRNA, suggests that common regulatory mechanisms may exist for inducing expression of hypha-specific genes. Initiation of transcription control is mediated by *cis*-acting sequences, termed gene response elements, that interact with DNA binding proteins to repress or activate transcription in response to environmental signals. Upstream Activating Sequence (UAS) and Upstream Regulatory Sequence (URS) elements serve to activate or repress gene expression through interactions with DNA binding proteins. Hypha-specific genes are expected to have both UAS and URS elements that are responsible for turning on genes in hyphal growth and repressing them during yeast growth. The presence of common *cis*-acting elements in different genes indicates that a mechanism for coordinate regulation of a set of genes that are appropriate for a given set of environmental conditions exists. Analysis and determination of the mechanisms regulating expression of these genes may also provide insight into the mechanism of the yeast/hypha transition in *C. albicans.*

Although *trans*-acting proteins involved in gene expression of defined hypha-specific genes have not been reported, white-phase specific factors have been identified for the yeast-specific gene *WH11,* which was discovered upon analysis of the white/opaque switching system of *C*. *albicans.* The function of regions upstream of *WH11* and *OP4* are the best analyzed and understood to date. A functional analysis of the *WH11* promoter region has led to the identification of upstream *cis*-acting regulatory sequences that form complexes with putative trans-acting proteins in cell extracts (Srikantha et al., 15(3) MOL. CELL. BIOL. 1797-805 (1995)), whereas a MADS box protein consensus binding site has been shown to be important for expression of *OP4*. Lockhart et al., 180(24) J. BACTERIOL. 6607-16 (1998). Further, the repression of *WH11* expression in *C*. *albicans* hyphae support the involvement of transcriptional control in the yeast/hypha transition. Importantly, the functional determinants in the *WH11* promoter were not found have sequence similarity to *cis*-acting regulatory regions from other organisms indicating that *C*. *albicans* probably has regulatory factors with features that are distinct from those of mammalian hosts and would therefore serve as suitable targets for development of antifungal strategies.

The mechanisms that control expression of hypha-specific genes may also regulate the bud/hypha transition. Work on *HWP1* has shown that *HWP1* expression always coincides with hypha production, suggesting that common mechanisms may regulate morphogenesis and hypha-specific gene expression. Therefore knowledge about dimorphism is relevant. Pseudohyphal (elongated yeasts) growth of *S. cerevisiae* was used as a model for pseudohyphal and hyphal growth of *C. albicans. STE12* of *S. cerevisiae,* the target of the pheromone response mitogen-activated protein kinase cascade and other upstream kinases, was found to be important for pseudohyphal growth of *S. cerevisiae.* Liu et al., 262(5140) SCIENCE 1741-44 (1993). Homologs of members of this pathway were similarly found to be important in pseudohyphal and hyphal growth of *C*. *albicans.* Kohler et al., 93(23) PROC. NATL. ACAD. SCI. USA 13223-28 (1996); Leberer et al., 93(23) PROC. NATL. ACAD. SCI. USA 13217-22 (1996); Liu et al., 262(5140) SCIENCE 1741-44 (1993). A *C. albicans STE12* homologue, *CPH1*, was also found to be necessary for production ofpseudohyphae of *C*. *albicans* on nitrogen poor solid media. Kohler & Fink, 266(5191) SCIENCE 1723-6 (1994). However, *CPH1* mutants still formed pseudohyphae and true hyphae when grown in liquid media and in response to serum. These results show that *CPH1* influences growth morphology in *C. albicans*, but, probably plays a limited role, and other unidentified factors are also required.

A second signal transduction pathway that is involved in pseudohyphal growth of *S. cerevisiae* is the cAMP/PKA pathway. Increasing the level of CAMP artificially by mutation in a phosphodiesterase, or by activating mutations of Ras1 protein GTPase or by adding exogenous cAMP enhances pseudohyphal formation. Gimeno et al., 68(6) CELL 1077-90 (1992); Lorenz & Heitman, 16(23) EMBO J. 7008-18 (1997); Ward et al., 15(12) MOL. CELL. BIOL. 6854-63 (1995). Mutations that decrease the levels of cellular cAMP result in decreased filamentation presumably due to the reduction of cAMP. Kubler et al., 272(33) J. BIOL. CHEM. 20321-3 (1997); Lorenz & Heitman, 16(23) EMBO J. 7008-18 (1997). The regulation of filamentation by cAMP levels also extends to other filamentuous fungi. Madhani & Fink, 8(9) TRENDS CELL. BIOL. 348-53 (1998). Indeed, a gene that is required for normal cAMP levels is also necessary for both dimorphism and *HWP1* production.

The MAPK and cAMP signaling pathways effect expression of the cell wall protein genes of *S. cerevisiae.* Both pathways affect the promoter of *FLO11*, whose gene product is a cell wall protein required for pseudohyphal growth. Rupp et al., 18(5) EMBO J. 1257-69 (1999). The *FLO11* promoter is unusually large (2.8 kb), and has at least four UAS and nine URS elements. Similarly, the intergenic region between *HWP1 and APL2* is also large (approximately 2.0 kb), suggesting that a conservation in the complexity required for regulation of filamentation genes may exist; *HWP1* probably also has multiple UAS and URS elements.

The *C. albicans HWP1* gene is likely under the control of the cAMP-dependent signalling pathway but not the MAPK pathway, in that *HWP1* expression was not altered in a *cph1* null mutant but was totally blocked in an *efg1* mutant. Sharkey et al., 184(17) J. BACTERIOL. 5273-9 (1999). Efglp is believed to be a transcriptional target of the cAMP dependent signaling pathway in *C. albicans* because of a putative proteinase K modification site. Efg1p is also necessary for formation of germ tubes and for virulence in an animal model. *HWP1* expression is also reduced in mutants lacking the *REF1* gene which encodes a different transcription factor that has not been associated with a particular signal transduction pathway. Ishii et al., 143(Pt 2) MICROBIOL. 429-35 (1997). Indeed, expression of hypha-specific genes and production of hyphae themselves may thus share common regulatory features.

The environmental cues that activate bud/hypha signalling cascades are unknown, but historical data strongly suggest that nitrogen regulatory circuits are involved. In many fungi, nitrogen utilization is important not only for the synthesis of essential metobolites, but also for interconversion between growth morphologies that are important for environmental adaptation. Spore germination in *Aspergillus* and *Rhizopus* is preferentially induced in poor nitrogen sources such as proline. Weber & Ogawa, 55 PHYTOPATH. 262-66 (1965); Yanagita, 26 ARCH. MICROBIOL. 329-44 (1957). In diploid strains of *S. cerevisiae,* nitrogen starvation or use of proline as a nitrogen source leads to production of pseudohyphal cells which invade solid agar, and are thought to be necessary for growth in the natural environment. Gimeno et al., 68(6) CELL 1077-90 (1992). In *C. albicans,* the use of proline or selected other amino acids induces production of true hyphae, as well as pseudohyphae from yeast forms. Dabrowa et al., 13(3) INFECT. IMMUN. 830-35 (1976); Holmes & Shepherd, 133(Pt II) J. GEN. MICROBIOL. 3219-28 (1987); Land et al., 11(5) INFECT. IMMUN. 1014-23 (1975). Morphologic variation in response to proline is also found in plant pathogenic fungi and is thought to be important for pathogenesis. Kulkami & Nickerson, 5 Exp. MYCOL. 145-54 (1981). Thus morphologic variation that accompanies the use of nitrogen sources is a common feature of fungal growth.

The control of nitrogen supply by prokaryotic and eukaryotic organisms is highly regulated. Fungi are able to utilize an array of compounds as nitrogen sources, and have evolved the capability to express different catabolic enzymes to make nitrogen available to the cell. Nitrogen metablism regulation has been well studied in *S. cerevisiae*, *Aspergillus nidulans*, and *Neurospora crassa.* When preferred sources of nitrogen such as ammonia, glutamine, and glutamate are not available or are in concentrations too low to support growth, the synthesis of pathway-specific catabolic enzymes and permeases are derepressed (53). Activating global regulatory genes in *Aspergillus* (*areA;* (CADDICK, MOLECULAR BIOLOGY OF FILIMENTOUS FUNGI 141-52 (1992); Kudla et al., 9(5) EMBO J. 1355-64 (1990)), *Neurospora* (*nit-2;* (Fu & Marzluf, 7(5) MOL. CELL. BIOL. 1691-96 (1987); Stewart & Vollmer, 46(2-3) GENE 291-95 (1986)), *Saccharomyces* (*gln-3;* Minehart & Magasanik, 11(12) MoL. CELL. BIOL. 6216-28 (1991)), and *Penicillum* (*nre;* Haas et al., 27(2) CURR. GENET. 150-58 (1995)) have been found that code for GATA-type zinc finger transcription factors which activate specific catabolic genes when preferred nitrogen sources are lacking: These regulatory proteins all have a conserved DNA binding domain which consists of a single Cys₂/Cys₂-type zinc finger motif with a central loop of 17 amino acid residues. Marzluf, 61(1) MOL. BIOL. REV. 17-32 (1997). The amino acid conservation in the DNA binding region is high among the different members of this GATA family. Mutations of conserved residues in the DNA binding domains of NIT2 or AREA lead to complete lack of DNA binding *in vitro* and are nonfunctional *in vivo.* Fu & Marzluf, 4(11) MOL. MICROBIOL. 1847-52 (1990).

URS elements may indeed mediate repression of filamentous growth in *C. albicans* strains. Strains that lack the *S. cerevisiae* repressor protein homologue, *TUP1* of *C. albicans* grow exclusively in filamentous form. Braun & Johnson, 277(5322) SCIENCE 105-09 (1997). Filamentous forms of *tup1* mutant cells range from short pseudohyphae to long thread-like structures resembling true hyphae depending on the environmental and media conditions. The *TUP1* gene of *C*. *albicans* was studied based on its role in *S*. *cerevisiae* where it serves as a pleiotropic repressor for many genes through protein-protein interactions with DNA binding proteins. Indeed, there may exist in yeast forms of *C*. *albicans* of DNA-binding repressor proteins that strongly regulate the bud/hypha transition.

*TUP1* may partially repress expression of hypha-specific genes. In a differential screen for genes that are expressed in *tup1* mutant cells, but not in *TUP1* parental cells, the mRNA for the hypha-specific gene, *HWP1*, was found. Sharkey et al. reported that *HWP1* was deprepressed in a *tup1* mutant. Sharkley et al. However, when *tup1* mutants were analyzed, surface HWP1 protein was not detected on hyphal surfaces under most culture conditions, suggesting that release from *TUP1* repression permits only partial *HWP1* expression and that additional proteins are required for normal levels and surface localization of hypha-specific gene products. Indeed, the upstream regions of hypha-specific genes of *C*. *albicans* may contain sites for proteins that interact with the *TUP1* gene product, or perhaps with Tup1 itself; however, interaction with Tup1 is less likely because *S. cerevisiae* Tup 1 protein is not a DNA binding protein, but functions through interactions with other DNA binding proteins. The partial derepression of *HWP1* in *tup1* mutants incidates that proteins other than *TUP1* repress *HWP1* in yeasts.

A powerful method for identifying UAS and URS is to fuse DNA upstream of a gene coding regions to a reporter gene whose protein level can be easily measured. URS elements are identified by increased reporter gene expression relative to the intact DNA upon deletion of the element. Conversely, UAS elements required for expression are identified by a loss of reporter gene activity upon deletion of the element. A second approach for identifying UAS elements is to design individual, overlapping bp sequence elements spanning the entire DNA sequence, and then look for activation of reporter gene expression relative to a simple promoter containing a TATA element. Guarente & Ptashne, 78(4) PROC. NATL. ACAD. SCI. USA 2199-203 (1981); Rupp et al. 18(5) EMBO J. 1257-69 (1999).

The *FLO11* gene upstream region, which is similar to *HWP1* in having an unusually large upstream region (Rupp et al. 18(5) EMBO J. 1257-69 (1999)), was studied using the *lacZ* reporter gene whereas the luciferase reporter gene was used to study regulatory regions in *C. albicans* genes that were under the control of switching. GUARENTE, THE MOLECULAR AND CELLULAR BIOLOGY OF THE YEAST SACCHAROMYCES CEREVISIAE (E. Jones, J. Pringle, and J. Broach ed.) (1992).

Green Fluorescent Protein (GFP) has proven to be a valuable marker of gene expression in studies employing optical microscopy in research in microbial pathogenesis. The advantages of GFP have been made available for studies in *C*. *albicans* by B. Cormack et al., who optimized all the GFP codons for expression in *C. albicans.* Cormack et al., 143(Pt 2) MICROBIOL. 303-11 (1997). GFP within *C. albacans* is readily visible by fluorescence microscopy and is easily quantified by flow cytometry. GFP in solution is also quantifiable by fluorometry. Kahn et al., 7(4) CURR. BIOL. R207-08 (1997). These advances indicate that GFP will be an excellent reporter for studies on inducible expression of HWP1 in *C*. *albicans*.

The transcriptional regulation of gene expression through *cis*-acting sequences is controlled by DNA binding proteins. Schleif, 241(4870) SCIENCE 1182-87 (1988). Evidence for the presence of unknown DNA binding proteins is provided by electrophoretic mobility shift assays, where proteins in crude nuclear extracts of cells growing in appropriate conditions are assayed for their ability to retard the migration of labeled UAS or URS elements in polyacrylamide gels. Mutations or deletions in the UAS are employed to verify the specificity of the interactions of DNA binding proteins with the UAS element and to map the most important nucleotides for DNA binding. The presence of unknown binding proteins to UAS elements provides the groundwork for cloning genes encoding regulatory proteins.

Several strategies have been successfully used to clone genes encoding proteins that bind to UAS elements. A particularly successful method is to use DNA affinity beads that consist of UAS elements that have incorporated biotinylated nucleotides to facilitate coupling to streptavidin beads. Gabrielsen & Huet, 218 METHODS ENZYMOL. 508-25 (1993). Nuclear extracts are then incubated with the beads to separate the desired DNA binding proteins from other proteins in the extract, followed by elution of the DNA binding protein from the beads, purification on SDS polyacrylamide gels and amino acid sequencing of HPLC-purified proteolytic fragments. The amino acid sequences are then used to design degenerate primers for amplification of a PCR product from cDNA to be used as a probe in library screening to obtain the DNA binding protein gene. Verification of the biological activity of the DNA binding protein is gained from creating null mutants in the gene and looking for loss of expression of the reporter gene under the control of the specific UAS elements used to identify and characterize the protein. The pattern of expression of the DNA binding protein gene itself, along with the predicted primary amino acid sequence, provide insight into regulatory mechanisms. Predictions about whether expression is mediated by transcription, or by protein modification, are possible. In the event that the DNA binding protein gene is regulated by transcription, its non-flanking regions provide material for identification of *cis-*acting regulatory regions as proposed for the primary target gene, *HWP1.*

One of the benefits of cloning gene(s) encoding proteins that bind to the promoter of a target gene like *HWP1* is that global gene regulatory networks can be readily sought. Target genes that are believed to be activated by the same mechanism will have reduced levels of mRNA in a null mutant lacking the DNA binding protein gene. For example, expression of sporulation specific genes of *S. cerevisiae* is regulated by the *IME1* gene. Deletion of *IME1* results in the loss of expression of an entire family of sporulation-specific genes. Mitchell, 58(1) MICROBIOL. REV. 56-70 (1994). Within the context of the present invention, all of the known hypha-specific genes will be assessed for expression in a null mutant of the DNA binding protein gene compared to cells having the gene, thereby revealing the importance of the DNA binding protein in regulating a set of genes.

Another aspect of the present invention is the identification of new genes that are regulated by hypha-specific controls, because such genes are likely to be important in virulence. The null mutant of the putative DNA binding protein genes serves as a tool to achieve this goal as well, for identification of genes with low message levels compared to mRNA from cells with the wild-type DNA binding protein gene. The ideal methodology for the identification of new genes is genome wide expression monitoring. DeRisi & Iyer, 11(1) CURR^{.} OPIN^{.} ONCOL. 76-79 (1999). The proven utility of genome wide expression monitoring in revealing previously unidentified genes that are up or down-regulated under any given environmental conditions has been demonstrated multiple times. For example, new sporulation-specific genes, and regulatory circuits were identified using genome-wide expression monitoring of the *S. cerevisiae* genome. Cox et al., 15(8) YEAST 703-13 (1999). Mammalian genes in fibroblasts that are regulated in the presence of serum have also been identified using genome wide expression monitoring with the available but incomplete mammalian genome sequences. Iyer et al., 283(5398) SCIENCE 83-87 (1999). In both cases, unexpected genes were identified underscoring the value of the technique. Similar studies are plausible using the *C. albicans* genome. The sequence of the *C. albicans* genome has been completed to five times coverage.

Given the high significance of the identification of new genes with potential importance in virulence in an organism where random mutagenesis approaches are not possible, exploration of alternatives other than genome wide expression monitoring may be warranted. Consequently, a "work-around" method that does not require the production of gene-specific probes may be utilized. Primers that hybridize to vector sequences are employed to amplify small genomic inserts that are robotically spotted on membranes to generate mini-arrays. This array method is being used for genome wide expression monitoring in *H. capsulatum.*

Opportunities for proliferation and invasion of mammalian hosts by *C. albicans* are continuing to increase. Because of the loss of the cellular component of the immune system, AIDS patients are susceptible to invasion of submucosal tissue by *C. albicans.* The frequency of candidal infections may also be a result of the prophylactic use of antibacterial drugs used in AIDS patients to minimize other opportunistic infections. Candidal infections increase in severity and recur more frequently as the immunodeficiency progresses. In non-AIDS patients, such as those undergoing organ transplantation, are neutropenic, or have debilitating diseases requiring advanced modalities of life support, mucosal and hematogenously disseminated candidiasis seriously threaten optimal treatment outcomes. While antifungal drugs can be effective, the increasing frequency of resistant strains of *C. albicans,* and the systemic side effects of the drugs prompts exploration of novel strategies to interrupt the sequence of events leading to disease and to expand the repertoire of antifungal drugs. Both goals require improved understanding of hypha-specific proteins of *C*. *albicans* in terms of their roles in pathogenicity and in the mechanisms that regulate their expression. By defining the molecular events leading to the expression of a pathogenically important adhesin, and through the identification of new genes that are co-regulated with *HWP1* in a putative global regulatory circuit, the present invention relates to new and novel ways to interfere with candidiasis. The long term medical benefits of this study may be the development of alternative or adjunctive therapies based on the knowledge expression of hypha-specific genes in *C. albicans.*

### Recombinant techniques

The DNA molecules of the present invention may be employed by recombinant techniques. Thus, for example, the DNA molecule sequence may be included in any one of a variety of expression vehicles, in particular vectors or plasmids for expressing such a polypeptide. Such vectors include chromosomal, nonchromosomal and synthetic DNA sequences, e.g., derivatives of SV40; bacterial plasmids; phage DNA; yeast plasmids; vectors derived from combinations of plasmids and phage DNA; viral DNA such as vaccinia, adenovirus, fowl pox virus, and pseudorabies. However, any other vector or plasmid may be used as long as they are replicable and viable in the host.

The appropriate DNA sequence may be inserted into the vector by a variety of procedures. In general, the DNA sequence is inserted into an appropriate restriction endonuclease sites by procedures known in the art. Such procedures and others are deemed to be within the scope of those skilled in the art.

The DNA sequence in the expression vector may be operatively linked to an appropriate expression control sequence(s) (promoter) to direct mRNA synthesis. As representative examples of such promoters, there may be mentioned: LTR or SV40 promoter, the *E. coli. lac* or *trp,* the phage lambda P_{L} promoter and other promoters known to control expression of genes in prokaryotic or eukaryotic cells or their viruses. The expression vector also contains a ribosome binding site for translation initiation and a transcription terminator. The vector may also include appropriate sequences for amplifying expression.

In addition, the expression vectors preferably may contain a gene to provide a phenotypic trait for selection of transformed host cells such as dihydrofolate reductase or neomycin resistance for eukaryotic cell culture, or such as tetracycline or ampicillin resistance in *E. coli.*

An embodiment of the invention comprises an isolated DNA molecule comprising the nucleotide sequence of the HWP gene promoter. This nucleotide sequence, or fragments or functional equivalents thereof, may be used to generate recombinant DNA molecules that direct the expression of the polypeptides of the present invention, or functionally active peptides or functional equivalents thereof, in appropriate host cells. Due to the degeneracy of the nucleotide coding sequence, other DNA sequences which encode substantially the same amino acid sequences as depicted, or analogs or fragments thereof, may be used in the practice of the invention for the cloning and expression of such a promoter. Such alterations include deletions, additions or substitutions of different nucleotide residues resulting in a sequence that encodes the same or a functionally equivalent gene product. The gene product may contain deletions, additions or substitutions of amino acid residues within the sequence, which result in a silent change thus producing a bioactive product. Such amino acid substitutions may be made on the basis of similarity in polarity, charge, solubility, hydrophobicity, hydrophilicity, the amphipathic nature of the residues involved and/or on the basis of crystallographic data. For example, negatively charged amino acids include aspartic acid and glutamic acid; positively charged amino acids include lysine and arginine; amino acids with uncharged polar head groups having similar hydrophilicity values include the following: leucine, isoleucine, valine; glycine, alanine; asparagine, glutamine; serine, threonine; phenylalanine, tyrosine.

Techniques well known to those skilled in the art for the isolation of DNA, generation of appropriate restriction fragments, construction of clones and libraries, and screening recombinants may be used. For a review of such techniques, see, for example, MANIATIS ET AL., MOLECULAR CLONING: A LABORATORY MANUAL, Chapters 1-18 (2^{nd} Ed., Cold Spring Harbor Laboratory 1989). Also, the 5' untranslated and coding regions of the nucleotide sequence could be altered to improve the translational efficiency of the mRNA. In addition, based on X-ray crystallographic data, sequence alterations could be undertaken to improve protein stability, e.g., introducing disulfide bridges at the appropriate positions, and/or deleting or replacing amino acids that are predicted to cause protein instability. These are only examples of modifications that can be engineered to produce a more active or stable protein, more protein, or even change the substrate specificity of the protein.

The vector containing the appropriate DNA sequence, as well as an appropriate promoter or control sequence, may be employed to transform an appropriate host to permit the host to express the polypeptides of the present invention. Representative examples of appropriate hosts include: bacterial cells, such as *E. coli*, *Salmonella typhimurium*, *Streptomyces;* fungal cells, such as yeast; insect cells, such as Drosophila S2 and Spodoptera Sf9; animal cells, such as CHO, COS or Bowes melanoma; adenoviruses; plant cells, etc.. The selection of an appropriate host and transformation technique within the scope of those skilled in the art.

More particularly, the present invention also includes recombinant constructs comprising one or more of the sequences of the present invention. The constructs may comprise a vector, such as a plasmid or viral vector, into which a sequence of the invention has been inserted, in a forward or reverse orientation. In a preferred aspect of this embodiment, the construct may further comprise regulatory sequences, including, for example, a promoter operably linked to the sequence. Large numbers of suitable vectors and promoters are known to those of skill in the art, and are commercially available. The following vectors are provided by way of example. Bacterial: pQE70, pQE60, pQE-9 (Qiagen), pBS, phagescript, psiX174, pBluescript SK, pBsKS, pNH8a, pNH16a, pNH18a, pNH46a (Stratagene); pTRC99A, pKK223-3, pKK233-3, pDR540, PRIT5 (Pharmacia). Eukaryotic: pWLneo, pSV2cat, pOG44, pXT1, pSG (Stratagene); pSVK3, pBPV, pMSG, PSVL (Pharmacia). However, any other plasmid or vector may be used as long as they are replicable and viable in the host.

In a further embodiment, the present invention relates to host cells containing the above-described construct. The host cell can be a higher eukaryotic cell, such as a mammalian cell, or a lower eukaryotic cell, such as a yeast cell, or the host cell can be a prokaryotic cell, such as a bacterial cell. The host cell preferably may secrete the recombinant protein. Introduction of the construct into the host cell can be effected by calcium phosphate transfection, DEAE-Dextran mediated transfection, or electroporation (L. DAVIS ET AL., BASIC METHODS IN MOLECULAR BIOLOGY, 1986)).

### Promoters

Suitable promoter regions can be selected from any desired gene using CAT (chloramphenicol transferase) vectors or other vectors with selectable markers. Two appropriate vectors are pKK232-8 and pCM7. Particular named bacterial promoters include laci, lacZ, T3, T7, gpt, lambda P_{R}, P_{L}and trp. Eukaryotic promoters include CMV immediate early, HSV thymidine kinase, early and late SV40, LTRs from retrovirus, and mouse metallothionein-I. Selection of the appropriate vector and promoter is well within the level of skill in the art. The constructs in host cells can be used in a conventional manner to produce the gene product encoded by the recombinant sequence.

### Enhancers

Transcription of a DNA encoding the polypeptides of the present invention by higher eukaryotes may be increased by inserting an enhancer sequence into the vector. Enhancers are cis-acting elements of DNA, usually from about 10 to 300 bp, that act on a promoter to increase its transcription. Examples include the SV40 enhancer on the late side of the replication origin (base pair 100 to 270), a cytomegalovirus early promoter enhancer, a polyoma enhancer on the late side of the replication origin, and adenovirus enhancers.

### Selectable markers

Generally, recombinant expression vectors will include origins of replication and selectable markers permitting transformation of the host cell, e.g., the ampicillin resistance gene of *E. coli* and *S. cerevisiae* TRP1 gene, and a promoter derived from a highly-expressed gene to direct transcription of a downstream structural sequence. Such promoters can be derived from operons encoding glycolytic enzymes such as 3-phosphoglycerate kinase (PGK), alpha factor, acid phosphatase, or heat shock proteins, among others. The heterologous structural sequence is preferably assembled in appropriate phase with translation, initiation and termination sequences, and preferably, a leader sequence capable of directing secretion of translated protein into the periplasmic space or extracellular medium. Optionally, the heterologous sequence can encode a fusion protein including an N-terminal identification peptide imparting desired characteristics, e.g., stabilization or simplified purification of expressed recombinant product.

### Expression vectors

Useful expression vectors for bacterial use may be constructed by inserting a structural DNA sequence encoding a desired protein together with suitable translation, initiation and termination signals in operable reading phase with a functional promoter. The vector may comprise one or more phenotypic selectable markers and an origin of replication to ensure maintenance of the vector and to, if desirable, provide amplification within the host. Suitable prokaryotic hosts for transformation include *E. coli, Bacillus subtilis, Salmonella typhimurium* and various species within the genera *Pseudomonas, Streptomyces,* and *Staphylococcus,* although others may also be employed as a matter of choice.

As a representative but nonlimiting example, useful expression vectors for bacterial use can comprise a selectable marker and bacterial origin of replication derived from commercially available plasmids comprising genetic elements of the well known cloning vector pBR322 (ATCC 37017). Such commercial vectors include, for example, pKK223-3 (Pharmacia Fine Chemicals, Uppsala, Sweden) and GEM1 (Promega Biotec, Madison, WI). These pBR322 backbone sections are combined with an appropriate promoter and the structural sequence to be expressed.

Following transformation of a suitable host strain and growth of the host strain to an appropriate cell density, the selected promoter may be de-repressed by appropriate means (e.g., temperature shift or chemical induction) and cells may be cultured for an additional period. Cells are typically harvested by centrifugation, disrupted by physical or chemical means, and the resulting crude extract retained for further purification.

Microbial cells employed in expression of proteins can be disrupted by any convenient method, including freeze-thaw cycling, sonication, mechanical disruption, or use of cell lysing agents. Various mammalian cell culture systems can also be employed to express recombinant polypeptides. Examples of mammalian expression systems include the COS-7 lines of monkey kidney fibroblasts, described by Gluzman and other cell lines capable of expressing a compatible vector, for example, the C127, 3T3, CHO, HeLa and BHK cell lines. Gluzman, 23(1) CELL 175-82 (1981). Mammalian expression vectors may comprise an origin of replication, a suitable promoter and enhancer, and also any necessary ribosome binding sites, polyadenylation sites, splice donor and acceptor sites, transcriptional termination sequences, and 5' flanking nontranscribed sequences. DNA sequences derived from the SV40 viral genome, for example, SV40 origin, early promoter, enhancer, splice, and polyadenylation sites may be used to provide the required nontranscribed genetic elements.

### Gene Therapy

The present invention also discloses the use of gene therapy applications, which will interfere with the transcription mediated by *cis* acting sequences of hyphal-specific genes. Gene therapy has been broadly defined as "the correction of a disease phenotype through the introduction of new genetic information into the affected organism." Roemer & Friedmann, 208(2) EUR. J. BIOCHEM. 211-25 (1992). Two basic approaches to gene therapy have evolved: (1) *ex vivo* gene therapy and (2) *in vivo* gene therapy. In *ex vivo* gene therapy, cells are removed from a subject and cultured *in vitro.* A functional replacement gene is introduced into the cells (transfection) *in vitro,* the modified cells are expanded in culture, and then reimplanted in the subject. These genetically modified, reimplanted cells are reported to secrete detectable levels of the transfected gene product *in situ.* Miller, 76(2) BLOOD 271-78 (1990); Selden et al., 317(17) NEW ENG. J. MED. 1067-76 (1987). The development of improved retroviral gene transfer methods (transduction) facilitates the transfer into and subsequent expression of genetic material by somatic cells. Cepko et al., 37(3) CELL 1053-62 (1984). Accordingly, retrovirus-mediated gene transfer has been used in clinical trials to mark autologous cells and as a way of treating genetic disease. Rosenberg et al., 323(9) NEW ENG. J. MED. 570-78 (1990); Anderson, 2 HUM. GENE THER. 99-100 (1991). Several *ex vivo* gene therapy studies in humans have been reported. Anderson, 256(5058) SCIENCE 808-13 (1992); Miller, 357(6378) NATURE 455-60 (1992).

In *in vivo* gene therapy, target cells are not removed from the subject. Rather, the transferred gene is introduced into cells of the recipient organism *in situ*, that is, within the recipient. *In vivo* gene therapy has been examined in several animal models. Felgner & Rhodes, 349(6307) NATURE 351-52 (1991). Publications have reported the feasibility of direct gene transfer *in situ* into organs and tissues such as muscle (Ferry et al., 88(19) PROC. NATL. ACAD. SCI. USA 8377-81 (1991); Quantin et al., 89(7) PROC. NATL. ACAD. SCI. USA 2581-84 (1992)), hematopoietic stem cells (Clapp et al., 78(4) BLOOD 1132-39 (1991)), the arterial wall (Nabel et al., 244(4910) SCIENCE 1342-44 (1989)), the nervous system (Price et al., 84(1) PROC. NATL. ACAD. SCI. USA 156-60 (1987)), and lung (Rosenfeld et al., 252(5004) SCIENCE 431-34 (1991)). Direct injection of DNA into skeletal muscle (Wolff et al., 247(4949 Pt 1) SCIENCE 1465-68 (1990)), heart muscle (Kitsis et al., 88(10) PROC. NATL. ACAD. SCI. USA 4138-42 (1991)) and injection of DNA-lipid complexes into the vasculature (Lim et al., 83(6) CIRCULATION 2007-11 (1991); Ledere et al., 90(3) J. CLIN. INVEST 936-44 (1992); Chapman et al., 71(1) CIRC. RES. 27-33 (1992)) also have been reported to yield a detectable expression level of the inserted gene product(s) *in vivo.*

Gene therapy efforts have been aimed at the identification of various cell types for transformation, including keratinocytes (Morgan et al., 237(4821) SCIENCE 1476-79 (1987)), fibroblasts (Palmer et al., 88(4) PROC. NATL. ACAD. SCI. USA 1330-34 (1991); Garver, Jr. et al., 237(4816) SCIENCE 762-64 (1987); International Patent Application PCT/US92/01890, having publication number WO 92/15676), lymphocytes (Reimann et al., 89(1) J. IMMUNOL. METHODS 93-101 (1986)), myoblasts (Barr & Leiden, 254(5037) SCIENCE 1507-09 (1991); Dai et al., 89(22) PROC. NATL. ACAD. SCI. USA 10892-5 (1992); Roman et al., 18(3) SOMAT. CELL MOL. GENET. 247-58 (1992)), smooth muscle cells (Lynch et al., 89(3) PROC. NATL. ACAD. SCI. USA 1138-42 (1992)), and epithelial cells (Nabel et al., 244(4910) SCIENCE 1342-4 (1989), International Patent Application PCT/US89/05575, having publication number WO 90/06997).

The delivery of an effective dose of a prophylactic or therapeutic agent *in situ* depends on the efficiency of transfection (or transduction) as well as the number of target cells. Epithelial cell-based gene therapy, in particular, involves a relatively small area available *in situ* for receiving genetically modified epithelial cells. The delivery of an effective dose of prophylactic or therapeutic agent *in situ* thus depends upon the total number of implanted epithelial cells.

In one embodiment of the invention, exogenous genetic material (e.g., a cDNA encoding a polypeptide of the present invention) is introduced into a syngeneic host cell *ex vivo* or *in vivo* by genetic transfer methods, such as transfection or transduction, to provide a genetically modified host cell. Various expression vectors (i.e., vehicles for facilitating delivery of exogenous genetic material into a target cell) are known to one skilled in the art.

Transfection refers to the insertion of nucleic acid into a mammalian host cell using physical or chemical methods. Several transfection techniques are known to those of ordinary skill in the art including: calcium phosphate DNA co-precipitation (METHODS IN MOLECULAR BIOLOGY, Vol. 7, *Gene Transfer and Expression Protocols,* Ed. E.J. Murray, Humana Press (1991)); DEAE-dextran; electroporation; cationic liposome-mediated transfection; and tungsten particle-facilitated microparticle bombardment. Johnston, 346(6286) NATURE 776-77 (1990). Strontium phosphate DNA co-precipitation is a preferred transfection method. Brash et al., 7(5) MOLEC. CELL. BIOL. 2031-34 (1987).

In contrast, transduction refers to the process of transferring nucleic acid into a cell using a DNA or RNA virus. A RNA virus (i.e., a retrovirus) for transferring a nucleic acid into a cell is referred to herein as a transducing chimeric retrovirus. Exogenous genetic material contained within the retrovirus is incorporated into the genome of the transduced host cell. A host cell that has been transduced with a chimeric DNA virus (e.g., an adenovirus carrying a cDNA encoding a therapeutic agent) will not have the exogenous genetic material incorporated into its genome, but will be capable of expressing the exogenous genetic material that is retained extrachromosomally within the cell.

Typically, the exogenous genetic material includes the heterologous gene (usually in the form of a cDNA comprising the exons coding for the therapeutic protein) together with a promoter to control transcription of the new gene. The promoter characteristically has a specific nucleotide sequence necessary to initiate transcription. Optionally, the exogenous genetic material further includes additional sequences (i.e., enhancers) required to obtain the desired gene transcription activity. For the purpose of this discussion an enhancer is simply any non-translated DNA sequence which works contiguous with the coding sequence (in *cis*) to change the basal transcription level dictated by the promoter. Preferably, the exogenous genetic material is introduced into the host cell genome immediately downstream from the promoter so that the promoter and coding sequence are operatively linked so as to permit transcription of the coding sequence. A preferred retroviral expression vector includes an exogenous promoter element to control transcription of the inserted exogenous gene. Such exogenous promoters include both constitutive and inducible promoters.

Naturally-occurring constitutive promoters control the expression of essential cell functions. As a result, a gene under the control of a constitutive promoter is expressed under all conditions of cell growth. Exemplary constitutive promoters include the promoters for the following genes which encode certain constitutive or housekeeping functions: hypoxanthine phosphoribosyl transferase (HPRT), dihydrofolate reductase (DHFR) (Scharfmann et al., 88(11) PROC. NATL. ACAD. SCI. USA 4626-30 (1991)), adenosine deaminase, phosphoglycerol kinase (PGK), pyruvate kinase, phosphoglycerol mutase, the beta -actin promoter (Ch'ng et al., 86(24) PROC. NATL. ACAD. SCI. USA 10006-10 (1989)), and other constitutive promoters known to those of skill in the art. In addition, many viral promoters function constitutively in eukaryotic cells. These include: the early and late promoters of SV40, the long terminal repeats (LTRs) of Moloney Leukemia Virus and other retroviruses, and the thymidine kinase promoter of Herpes Simplex Virus, among many others. Accordingly, any such constitutive promoters can be used to control transcription of a heterologous gene insert.

Genes that are under the control of inducible promoters are expressed only or to a greater degree, in the presence of an inducing agent, (e.g., transcription under control of the metallothionein promoter is greatly increased in presence of certain metal ions). Inducible promoters include responsive elements (REs) which stimulate transcription when their inducing factors are bound. For example, there are REs for serum factors, steroid hormones, retinoic acid and cyclic AMP. Promoters containing a particular RE can be chosen in order to obtain an inducible response, and in some cases, the RE itself may be attached to a different promoter, thereby conferring inducibility to the recombinant gene. Thus, by selecting the appropriate promoter (constitutive versus inducible; strong versus weak), it is possible to control both the existence and level of expression of a therapeutic agent in the genetically modified host cell. If the gene encoding the prophylactic or therapeutic agent is under the control of an inducible promoter, delivery of the agent *in situ* is triggered by exposing the genetically modified cell *in situ* to conditions for permitting transcription of the prophylactic or therapeutic agent, e.g., by intraperitoneal injection of specific inducers of the inducible promoters which control transcription of the agent. For example, *in situ* expression by genetically modified host cells of a therapeutic agent encoded by a gene under the control of the metallothionein promoter, is enhanced by contacting the genetically modified cells with a solution containing the appropriate (i.e., inducing) metal ions *in situ.*

Accordingly, the amount of therapeutic agent that is delivered *in situ* is regulated by controlling such factors as: (1) the nature of the promoter used to direct transcription of the inserted gene (i.e., whether the promoter is constitutive or inducible, strong or weak); (2) the number of copies of the exogenous gene that are inserted into the host cell; (3) the number of transduced/transfected host cells that are administered (e.g., implanted) to the patient; (4) the size of the implant (e.g., graft or encapsulated expression system); (5) the number of implants; (6) the length of time the transduced/transfected cells or implants are left in place; and (7) the production rate of the prophylactic or therapeutic agent by the genetically modified host cell. Selection and optimization of these factors for delivery of an effective dose of a particular prophylactic or therapeutic agent is deemed to be within the scope of one of skill in the art, taking into account the above-disclosed factors and the clinical profile of the patient.

In addition to at least one promoter and at least one heterologous nucleic acid encoding the prophylactic or therapeutic agent, the expression vector preferably includes a selection gene, for example, a neomycin resistance gene, for facilitating selection of host cells that have been transfected or transduced with the expression vector. Alternatively, the host cells are transfected with two or more expression vectors, at least one vector containing the gene(s) encoding the prophylactic or therapeutic agent(s), the other vector containing a selection gene. The selection of a suitable promoter, enhancer, selection gene and/or signal sequence is deemed to be within the scope of one skilled in the art.

The prophylactic or therapeutic agent can be targeted for delivery to an extracellular, intracellular or membrane location. If it is desirable for the gene product to be secreted from the host cells, the expression vector is designed to include an appropriate secretion signal sequence for secreting the therapeutic gene product from the cell to the extracellular milieu. If it is desirable for the gene product to be retained within the host cell, this secretion signal sequence is omitted. In a similar manner, the expression vector can be constructed to include retention signal sequences for anchoring the prophylactic or therapeutic agent within the host cell plasma membrane. For example, membrane proteins have hydrophobic transmembrane regions that stop translocation of the protein in the membrane and do not allow the protein to be secreted. The construction of an expression vector including signal sequences for targeting a gene product to a particular location is deemed to be within the scope of one of skill in the art.

In an embodiment, vectors for mammalian host cell gene therapy are viruses, more preferably replication-deficient viruses (described in detail below). Exemplary viral vectors are derived from: Harvey Sarcoma virus; ROUS Sarcoma virus, MPSV, Moloney murine leukemia virus and DNA viruses (*e.g.*, adenovirus). TEMIN, GENE TRANSFER 149-187 (Kucherlapati, R., ed.) (1986).

Replication-deficient retroviruses are capable of directing synthesis of virion proteins, but are incapable of making infectious particles. Accordingly, these genetically altered retroviral expression vectors have general utility for high-efficiency transduction of genes in cultured cells, and specific utility for use in the methods of the present invention. Such retroviruses further have utility for the efficient transduction of genes into host cells *in vivo.* Retroviruses have been used extensively for transferring genetic material into cells. Standard protocols for producing replication-deficient retroviruses (including the steps of incorporation of exogenous genetic material into a plasmid, transfection of a packaging cell line with plasmid, production of recombinant retroviruses by the packaging cell line, collection of viral particles from tissue culture media, and infection of the target cells with the viral particles) are provided in KRIEGLER, GENE TRANSFER AND EXPRESSION, A LABORATORY MANUAL (Murray, E.J., ed.) (1990).

The major advantage of using retroviruses for gene therapy is that the viruses insert the gene encoding the therapeutic agent into the host cell genome, thereby permitting the exogenous genetic material to be passed on to the progeny of the cell when it divides. In addition, gene promoter sequences in the LTR region have been reported to enhance expression of an inserted coding sequence in a variety of cell types. *See e.g.,* Hilberg et al., 84(15) PROC. NATL. ACAD. SCI. USA 5232-36 (1987); Holland et al., 84(23) PROC. NATL. ACAD. SCI. USA 8662-66 (1987); Valerio et al., 84(2) GENE 419-27 (1989). *In vivo* gene therapy using replication-deficient retroviral vectors to deliver a therapeutically effective amount of a therapeutic agent can be efficacious if the efficiency of transduction is high and/or the number of target cells available for transduction is high.

Yet another viral candidate useful as an expression vector for transformation of mammalian host cells is the adenovirus, a double-stranded DNA virus. The adenovirus is frequently responsible for respiratory tract infections in humans and thus appears to have an avidity for the epithelium of the respiratory tract. STRAUS, THE ADENOVIRUS 451-496 (H.S. Ginsberg, ed.) (1984). Moreover, the adenovirus is infective in a wide range of cell types, including, for example, muscle and epithelial cells. Larrick & Burck, GENE THERAPY. APPLICATION OF MOLECULAR BIOLOGY 71-104 (1991). The adenovirus also has been used as an expression vector in muscle cells *in vivo.* Quantin et al., 89(7) PROC. NATL. ACAD. SCI. USA 2581 (1992).

Like the retrovirus, the adenovirus genome is adaptable for use as an expression vector for gene therapy, i.e., by removing the genetic information that controls production of the virus itself. Rosenfeld et al., 252(5004) SCIENCE 431-34 (1991). Because the adenovirus functions in an extrachromosomal fashion, the recombinant adenovirus does not have the theoretical problem of insertional mutagenesis.

Thus, as will be apparent to one skilled in the art, a variety of suitable viral expression vectors are available for transferring exogenous genetic material into mammalian host cells. The selection of an appropriate expression vector to express an agent for the identification, prevention or treatment of microbial infection amenable to gene replacement therapy and the optimization of the conditions for insertion of the selected expression vector into the cell are within the scope of one of skill in the art without the need for undue experimentation.

In an alternative embodiment, the expression vector is in the form of a plasmid, which is transferred into the target host cells by one of a variety of methods: physical (e.g., microinjection (Capecchi, 22(2 Pt 2) CELL 479-88 (1980)), electroporation (Andreason & Evans, 6(7) BIOTECHNIQUES 650-60 (1988)), scrape loading, microparticle bombardment (Johnston, 346(6286) NATURE 776-77 (1990)) or by cellular uptake as a chemical complex (e.g., calcium or strontium co-precipitation, complexation with lipid, complexation with ligand) (METHODS IN MOLECULAR BIOLOGY, Vol. 7, GENE TRANSFER AND EXPRESSION PROTOCOLS, (E. J. Murray, ed.) (1991)). Several commercial products are available for cationic liposome complexation including Lipofectin^{™} (Life Technologies, Inc., Gaithersburg, MD) (Felgner et al., 84(21) PROC. NATL. ACAD. SCI. USA 7413-17 (1987)) and Transfectam^{™} (ProMega, Madison, Wis.). Behr et al., 86(18) PROC. NATL. ACAD. SCI. USA 6982-86 (1989); Loeffler et al., 54(5) J. NEUROCHEM. 1812-15 (1990). However, the efficiency of transfection by these methods is highly dependent on the nature of the target cell and accordingly, the conditions for optimal transfection of nucleic acids into host cells using the above-mentioned procedures must be optimized. Such optimization is within the scope of one of skill in the art.

In an embodiment, the preparation of genetically modified host cells contains an amount of cells sufficient to deliver a prophylactically or therapeutically effective dose of a substrate for mammalian transglutaminases of the present invention to the recipient *in situ*. The determination of an effective dose of the prophylactic or therapeutic agent for a known microbial infection is within the scope of one of skill in the art. Thus, in determining the effective dose, the skilled artisan would consider the condition of the patient, the severity of the condition, as well as the results of clinical studies of the prophylactic or therapeutic agent being administered.

If the genetically modified host cells are not already present in a pharmaceutically acceptable carrier, they are placed in such a carrier prior to administration to the recipient. Such pharmaceutically acceptable carriers include, for example, isotonic saline and other buffers as appropriate to the patient and therapy. The genetically modified cells are administered by, for example, intraperitoneal injecting or implanting the cells or a graft or capsule containing the cells in a host cell-compatible site of the recipient. As used herein, host cell-compatible site refers to a structure, cavity or fluid of the recipient into which the genetically modified cell(s), host cell graft, or encapsulated host cell expression system can be implanted, without triggering adverse physiological consequences. Representative host cell-compatible sites include, for example, the peritoneal, pleural and pericardial cavities. Preferably, the host cell-compatible site communicates with the lymphatic system, thereby enabling delivery of the therapeutic agent to the vascular system.

In one embodiment, the host cell-compatible site may be denuded prior to implanting the cells. Exemplary denuding methods include but are not limited to: (1) injection of distilled water into the site (e.g., the peritoneal cavity) for 20 minutes, followed by scraping off a portion of the epithelial layer; (2) injection of 0.1 % buffered trypsin for 20 minutes followed by scraping; (3) removal of epithelial cells by gentle scraping with a cell scraper and (4) touching a piece of Gelfilm (Upjohn, Kalamazoo, MI) to the endothelium.

The genetically modified host cells are implanted in a host cell-compatible site, alone or in combination with other genetically modified host cells. Thus, the instant invention embraces a method for modifying the epithelial system of a recipient by using a mixture of genetically modified host cells, such that a first modified cell expresses a first prophylactic or therapeutic agent of the present invention and a second modified cell expresses a second prophylactic or therapeutic agent. Other genetically modified cell types (e.g., hepatocytes, smooth muscle cells, fibroblasts, glial cells, mesothelial cells or keratinocytes) can be added, together with the genetically altered epithelial cells, to produce expression of a complex set of introduced genes. Moreover, more than one recombinant gene can be introduced into each genetically modified cell on the same or different vectors, thereby allowing the expression of multiple prophylactic or therapeutic agents of the present invention by a single cell.

The instant invention further embraces an epithelial cell graft. The graft comprises a plurality of the above-described genetically modified cells attached to a support that is suitable for implantation into a mammalian recipient, preferably into the oral cavity. The support can be formed of a natural or synthetic material. According to another aspect of the invention, an encapsulated host cell expression system is provided. The encapsulated system includes a capsule suitable for implantation into a mammalian recipient and a plurality of the above-described genetically modified host cells contained therein. The capsule can be formed of a synthetic or naturally-occurring material. The formulation of such capsules is known to one of ordinary skill in the art. In contrast to the host cells that are directly implanted into the mammalian recipient (i.e., implanted in a manner such that the genetically modified cells are in direct physical contact with the host cell-compatible site), the encapsulated cells remain isolated (i.e., not in direct physical contact with the site) following implantation. Thus, the encapsulated host cell system is not limited to a capsule including genetically-modified non-immortalized host cells, but may contain genetically modified immortalized host cells.

### Polypeptides

As used herein, polypeptide refers to a linear series of amino acid residues connected to one another by peptide bonds between the alpha-amino groups and carboxy groups of adjacent amino acid residues. Additional covalent bonds between portions of the peptide are also present to restrain the conformation of the molecule, such as amide and disulfide bonds. When used herein, protein also refers to a linear series of amino acid residues connected one to the other as in a peptide. The term synthetic peptide means a chemically derived chain of amino acid residues linked together by peptide bonds that is free of naturally occurring proteins and fragments thereof.

Polypeptides of the present invention may include any analog, fragment or chemical derivative of the polypeptides capable of inhibiting transglutaminase-mediated microbial interaction with a mammalian host. Polypeptides thus may include soluble peptides, Ig-tailed fusion peptides, members of random peptide libraries (*see, e.g.,* Lam et al., 354(6348) NATURE 82-84 (1991); Houghten et al., 354(6348) NATURE 84-86 (1991)), combinatorial chemistry-derived molecular library made of D-and/or L- configuration amino acids, and phosphopeptides (including members of random or partially degenerate, directed phosphopeptide libraries, see, e.g., Songyang et al., 72(5) CELL 767-78(1993)).

Such polypeptides may include those derived from mammalian transglutaminases and those derived from mammalian transglutaminase substrates. The term analog refers to any polypeptide having an amino acid sequence, in comparison to the amino acid sequences of the polypeptides of the present invention, in which one or more amino acids have been substituted with other amino acids; where the substituted amino acids allow or require the polypeptide to assume the equilibrium conformation of the domain of the parent protein. Often, cysteine, lysine and glutamic acid will be used for their side chains which can form covalent linkages to restrict the conformation of a peptide.

The term analog shall also include any polypeptide which has one or more amino acids deleted from or added to an amino acid sequence of a mammalian transglutaminase substrate, but which still retains inhibitory activity against transglutaminase-mediated microbial interaction with a mammalian host. The term fragment shall refer to any shorter version of the polypeptides, wherein the fragment is capable of inhibiting transglutaminase-mediated microbial interaction with a mammalian host.

Of course, the present polypeptides may also be prepared by recombinant DNA techniques. The present invention also relates to vectors comprising DNA molecules of the present invention, host cells which are genetically engineered with vectors of the invention and the production of polypeptides of the invention by recombinant techniques. Host cells may be genetically engineered (transduced or transformed or transfected) with the vectors of this invention which may be, for example, a cloning vector or an expression vector. The vector may be, for example, in the form of a plasmid, a viral particle, a phage, etc. The engineered host cells can be cultured in conventional nutrient media modified as appropriate for activating promoters, selecting transformants or amplifying genes. The culture conditions, such as temperature, pH and the like, are preferably those previously used with the host cell selected for expression, and will be apparent to the skilled artisan. Mature proteins can be expressed in mammalian cells, yeast, bacteria, or other cells under the control of appropriate promoters. Cell-free translation systems can also be employed to produce such proteins using RNAs derived from the DNA constructs of the present invention. Appropriate cloning and expression vectors for use with prokaryotic and eukaryotic hosts are described by Sambrook et al., *supra.*

The polypeptides of the present invention may be recovered and purified from recombinant cell cultures by methods used heretofore, including ammonium sulfate or ethanol precipitation, acid extraction, anion or cation exchange chromatography, phosphocellulose chromatography, hydrophobic interaction chromatography, affinity chromatography, hydroxyapatite chromatography and lectin chromatography. Protein refolding steps can be used, as necessary, in completing configuration of the mature protein. Finally, high performance liquid chromatography (HPLC) can be employed for final purification steps.

The polypeptides of the present invention may be a naturally purified product, or a product of chemical synthetic-procedures, or produced by recombinant techniques from a prokaryotic or eukaryotic host (for example, by bacterial, yeast, higher plant, insect and mammalian cells in culture). Depending upon the host employed in a recombinant production procedure, the polypeptides of the present invention may be glycosylated with mammalian or other eukaryotic carbohydrates or may be non-glycosylated. Polypeptides of the invention may also include an initial methionine amino acid residue.

Any peptide of the present invention may be used in the form of a pharmaceutically acceptable salt. Suitable acids which are capable of forming salts with the peptides of the present invention include inorganic acids such as hydrochloric acid, hydrobromic acid, perchloric acid, nitric acid, thiocyanic acid, sulfuric acid, phosphoric acid and the like; and organic acids such as formic acid, acetic acid, propionic acid, glycolic acid, lactic acid, pyruvic acid, oxalic acid, malonic acid, succinic acid, maleic acid, fumaric acid, anthranilic acid, cinnamic acid, naphthalene sulfonic acid, sulfanilic acid, and the like.

Suitable bases capable of forming salts with the peptides of the present invention include inorganic bases such as sodium hydroxide, ammonium hydroxide, potassium hydroxide and the like; and organic bases such as mono-, di- and tri-alkyl and aryl amines (e.g., triethylamine, diisopropyl amine, methyl amine, dimethyl amine and the like) and optionally substituted ethanolamines (e.g., ethanolamine, diethanolamine and the like).

For use in a method of identification, prevention or treatment, such as the identification, prevention or treatment of infection of a mammalian host by a microorganism, the polypeptides of the present invention may be present in a pharmaceutical composition in admixture with a pharmaceutically acceptable sterile vehicle. The pharmaceutical composition may be compounded according to conventional pharmaceutical formulation techniques.

The vehicle may take a wide variety of forms depending on the form of preparation desired for administration, e.g., sublingual, rectal, nasal, oral or parenteral. Compositions for oral dosage form may include any of the usual pharmaceutical media, such as, for example, water, oils, alcohols, flavoring agents, preservatives, coloring agents and the like in the case of oral liquid preparations (e.g., suspensions, elixirs and solutions) or carriers such as starches, sugars, diluents, granulating agents, lubricants, binders, disintegrating agents and the like in the case of oral solid preparations (e.g., powders, capsules and tablets). Controlled release forms may also be used. Because of their ease in administration, tablets and capsules represent an advantageous oral dosage unit form, in which case solid pharmaceutical carriers may be employed. If desired, tablets may be sugar coated or enteric coated by standard techniques.

For compositions to be administered parenterally, the carrier will usually comprise sterile water, although other ingredients to aid solubility or for preservation purposes may be included. Injectable suspensions may also be prepared, in which case appropriate liquid carriers, suspending agents and the like may be employed. The parenteral routes of administration may be intravenous injection, intramuscular injection or subcutaneous injection.

For intravenous administration, the polypeptides may be dissolved in an appropriate intravenous delivery vehicle containing physiologically compatible substances such as sodium chloride, glycine and the like, having a buffered pH compatible with physiologic conditions. Such intravenous delivery vehicles are known to those skilled in the art.

The polypeptides of the invention may be administered to subjects where alteration of the hypha-specific gene expression is desired. The peptides may be administered by any convenient means. Oral administration is presently contemplated as a preferred administration route. The amount administered will depend on the activity of the particular compound administered, which may readily be determined by those of ordinary skill in the art.

### Antibodies

Antibodies of the present invention may include any polyclonal, monoclonal, humanized, anti-idiotypic, chimeric or single chain antibodies, and FAb, F(ab')₂ and FAb expression library fragments, and epitope-binding fragments thereof, specific to the polypeptides of the present invention.

Another embodiment of the present invention discloses a monoclonal antibody to the polypeptides of the present invention (or an antigenic portion thereof), which may be produced by methods recognized in the art, including the formation of monoclonal antibody-producing hybridomas. Kohler & Milstein, 256(5517) NATURE 495-97 (1975); Kohler & Milstein 6(7) EUR. J. IMMUNOL. 511-19 (1976).

In an embodiment of the invention, purified polypeptides of the present invention (or an antigenic portion thereof) can be used as an antigen or immunogen. In addition, microorganisms expressing HWP1 promoter regions, protein or polypeptide fragments thereof also represent potential antigens or sources of antigen with which to immunize animals to obtain somatic cells for fusion. Somatic cells with the potential for producing antibody and, in particular, B lymphocytes, are suitable for fusion with a B-cell myeloma line. Those antibody-producing cells that are in the dividing plasmablast stage fuse preferentially. Somatic cells may be derived from the lymph nodes, spleens and peripheral blood of primed animals and the lymphatic cells of choice depending to a large extent on their empirical usefulness in the particular fusion system. Once-primed or hyperimmunized animals can be used as a source of antibody-producing lymphocytes. Mouse lymphocytes give a higher percentage of stable fusions with mouse myeloma lines. However, the use of rat, rabbit, and frog cells is also possible. Alternatively, human somatic cells capable of producing antibody, specifically B lymphocytes, are suitable for fusion with myeloma cell lines. While B lymphocytes from biopsied spleens or lymph nodes of individual may be used, the more easily accessible peripheral blood B lymphocytes are preferred. The lymphocytes may be derived from patients with diagnosed carcinomas.

Specialized myeloma cell lines have been developed from lymphocyte tumors for use in hybridoma-producing fusion procedures. Kohler, & Milstein, 6(7) EUR. J. IMMUNOL. 511-19 (1976); Schulman et al., 276(5685) NATURE 269-70 (1978). Examples of myeloma cell lines that may be used for the production of fused cell hybrids include X63-Ag8, NSI-Ag4/1, MPCII-45.6TG1.7, C63-Ag8.653, Sp2/0-Ag14, FO, and S194/5XX0.BU.1, all derived from mice; 210.RCY3.Ag1.2.3, U-226AR, and GM1500GTGAL2, all derived from rats; and U-226AR and GM1500GTGAL2, derived from humans, (G.J. Hammerling, U. Hammerling, and J.F. Kearney (editors), *Monoclonal Antibodies and T-cell Hybridomas* in: J.L. Turk (editor) RESEARCH MONOGRAPHS IN IMMUNOLOGY, Vol. 3, Elsevier/North Holland Biomedical Press, NY (1981)).

Methods for generating hybrids of antibody-producing spleen or lymph node cells and myeloma cells usually comprise mixing somatic cells with myeloma cells in a 2:1 proportion (though the proportion may vary from about 20:1 to about 1:1), respectively, in the presence of an agent or agents (chemical or electrical) that promote the fusion of cell membranes. It is often preferred that the same species of animal serve as the source of the somatic and myeloma cells used in the fusion procedure. Fusion methods have been described by Kohler and Milstein (Kohler & Milstein, 256(5517) NATURE 495-97 (1975); Kohler & Milstein, 6 EUR. J. IMMUNOL. 511-19 (1976)), and by Gefter et al. (3 SOMATIC CELL GENET. 231-36 (1977)). The fusion-promotion agents used by those investigators were Sendai virus and polyethylene glycol (PEG), respectively.

The monoclonal antibodies of this invention can be used as probes in detecting discrete antigens expressed by microorganisms. The expression or lack of expression of these antigens can provide clinically exploitable information that is not apparent after standard histopathological evaluations. It may thus be possible to correlate the immuno-phenotypes of individual microorganisms with various aspects of microbial-mammalian host interaction and responsiveness to certain types of therapies, thereby establishing important classifications of prognosis.

The antibodies may also be used to detect drug resistance in microorganisms. For example, drug resistant *C. albicans* can make hyphae (germ tubes) in the presence of drug, but susceptible strains cannot. *See,* Gallagher et al., 138 J. GEN. MICROBIOL. 1901-11 (1992). Because HWP1 is a marker of hyphae formation, the detection of the presence or absence of HWP1 may be useful in the monitoring of drug resistance in *C. albicans.*

The use of the monoclonal antibodies described herein can be extended to the screening of human biological fluids for the presence of the specific antigenic determinant recognized. *In vitro* immunoserological evaluation of sera withdrawn from patients thereby permits non-invasive diagnosis of microbial infection. By way of illustration, human fluids, such as pleural fluids or lymph, can be taken from a patient and assayed for the specific epitope, either as released antigen or membrane-bound on cells in the sample fluid, using monoclonal antibodies against the polypeptides of the present invention in standard radioimmunoassays or enzyme-linked immunoassays known in the art or competitive binding enzyme-linked immunoassays.

The monoclonal antibodies of this invention are potentially useful for targeting microbial infection *in vivo.* They can therefore be used in humans for localization and monitoring of the microbial infection. For this application, it is preferable to use purified monoclonal antibodies. Purification of monoclonal antibodies for human administration by ammonium sulfate or sodium sulfate precipitation followed by dialysis against saline and filtration sterilization has been described. Miller et al., HYBRIDOMAS IN CANCER DIAGNOSIS AND THERAPY 134 (1982).

Alternatively, immunoaffinity chromatography techniques may be used to purify the monoclonal antibodies. The purified monoclonal antibodies can be labeled with radioactive compounds, for instance, radioactive iodine, and administered to a patient intravenously. After localization of the antibodies at the infection site, they can be detected by emission tomographical and radionuclear scanning techniques, thereby pinpointing the location of the infection. Experimental radioimmunodetection with monoclonal antibodies may occur by external scintigraphy.

Passive monoclonal serotherapy may be a potential use for the monoclonal antibodies of this invention. By way of illustration, purified anti-HWP1 monoclonal antibody is dissolved in an appropriate carrier, e.g., saline, with or without human albumin, at an appropriate dosage and is administered to a patient. The monoclonal antibodies are preferably administered intravenously, e.g., by continuous intravenous infusion over several hours, as in Miller et al.. Infusions can be administered over a period of weeks during which the anti-microbial effects are monitored.

In an alternate embodiment, the antibodies described herein are used to stimulate the production of corresponding anti-idiotypic antibodies. In brief, anti-idiotypic antibodies, or antiidiotypes are antibodies directed against the antigen combining region or variable region (idiotype) of another antibody. Based on Jerne's network model of idiotypic relationships (Jerne, 125 ANN. IMMUNOL. 373 (1974); Jerne et al., 1 EMBO 234 (1982)), immunization with an antibody molecule expressing a paratope (antigen-combining site) for a given antigen should produce a group of anti-antibodies, some of which share with the antigen a complementary structure to the paratope. Immunization with a subpopulation of antiidiotypic antibodies should in turn produce a subpopulation of antiidiotypic antibodies which bind the initial antigen. Thus, the administration of the monoclonal antibodies of the present invention may result in a modification of the host's anti-microbial immune response, as the consequence of the formation of anti-idiotypic antibodies which may develop during therapy with the monoclonals.

The monoclonal antibodies of this invention can be used in conjunction with a broad spectrum of pharmaceutical or cytotoxic agents that selectively affect a microorganism over the mammalian host. The methods used for binding the cytotoxic agents to the monoclonal antibody molecule can involve either non-covalent or covalent linkages. Since non-covalent bonds are more likely to be broken before the antibody complex reaches the target site, covalent linkages are preferred. For instance, carbodiimide can be used to link carboxy groups of the pharmaceutical agent to amino groups of the antibody molecule. Bifunctional agents such as dialdehydes or imidoesters can be used to link the amino group of a drug to amino groups of the antibody molecule. The Schiff base reaction can be used to link drugs to antibody molecules. This method involves the periodate oxidation of a drug or cytotoxic agent that contains a glycol or hydroxy group, thus forming an aldehyde that is then reacted with the antibody molecule. Attachment occurs via formation of a Schiff base with amino groups of the antibody molecule. Additionally, drugs with reactive sulfhydryl groups have been coupled to antibody molecules.

Another embodiment of the invention relates to a diagnostic kit for detecting a microorganism expressing a protein. The diagnostic kit may further comprise, where necessary, other components of the signal producing system, including agents for reducing background interference, control reagents, or an apparatus, container or other solid support for conducting the test. The binding of antibody to the target can be detected by well known methods, including radiation (e.g., use of a radioactive nucleotide), colorimetry (e.g., use of an enzyme that can cause a color change in a substrate), fluorescence (e.g., use of a dye such as propidium iodide, fluorescein, or rhodamine), and luminescence (e.g., use of an alkaline phosphatase substrate that releases photons upon cleavage or luciferin). Detection can be qualitative or quantitative.

The invention has been disclosed broadly and illustrated in reference to representative embodiments described above. Those skilled in the art will recognize that various modifications can be made to the present invention without departing from the spirit and scope thereof.

Without further elaboration, it is believed that one skilled in the art, using the preceding description, can utilize the present invention to the fullest extent. The following examples are illustrative only, and not limiting of the remainder of the disclosure in any way whatsoever.

### EXAMPLES

### Example 1: Role of HWP1 in the formation of candidiasis

### I. Structural features of the gene product of HWP1.

The complete *HWP1* gene was obtained by screening a *C*. *albicans* genomic library with cDNA which had been isolated following immunoscreening of a germ tube cDNA library with hypha-specific antibodies. Staab et al. (1996).

*HWP1* encodes a protein of 634 amino acids with an N-terminal antigenic domain that is more hydrophilic, more acidic, more likely to be present on the protein surface and has a higher antigenic index than the remainder of HWP1. In contrast to the uniqueness of the antigenic domain that was previously described, the central and C-terminal regions of HWP1 shares features that have been found in other yeast cell wall proteins, including a high percentage of serine and threonine residues that leads to extensive *O*-linked carbohydrate modification. The two serine/threonine-rich regions (from ILE₁₈₈ to CYS₃₈₉, and from THR₃₉₀ to GLU₆₁₂) are distinguishible by their mole percent of these two amino acids. In the first and second regions respectively, 54% and 30% of the residues are serine or threonine. The different content of serine and threonine residues may reflect distinct functions for each region. The second region is also rich in alanine and glutamine (20% of residues) and has a higher content of proline residues than the first region. The discovery of an abrupt increase in serine and threonine residues at the C-terminal boundary of the antigenic domain of HWP1 suggests that the "lollipop on a stick" model, in which a C-terminal domain rich in hydroxy amino acids serves to extend a binding domain above the cell surface, may help explain the exposure of the antigenic domain of HWP1 at the cell surface. Jentoft, 15 TR. BIOCHEM. SCI. 291-94 (1990). The absence of glycosylation sites resulting in a lack of carbohydrate masking in the *N*-terminal repetitive domain strongly suggest that this peptide sequence is important for interactions with host surfaces.

The first serine/threonine region shows similarity (BLASTP algorithm, Altschul et al., 215 J. MOL. BIOL. 403-10 (1990)) to mucins from different species because of small stretches of threonine residues in HWP1, while the second serine/threonine region did not show similarity to proteins in the databanks. Two 29 amino acid direct repeats separated by 5 residues were found in this latter region. The amino acid repeats were identical except for a conserved change of alanine to valine at position 22 within the repeat. In addition to multiple sites for *O*-linked carbohydrate addition, three potential *N*-glycosylation sites were identified at amino acid positions 241, 286 and 601, all of which were ASN-Xaa-SER. Finally, the hydrophobic C terminus in HWP1 is similar to other yeast cell membrane or cell wall proteins whose maturation involves transfer to a glycosylphosphatidylinositol (GPI) anchor.

HWP1 is acidic, having 63 negative charges at neutral pH, and an isoelectric point of 3.37, which is expected to contribute to the strong negative charge of surfaces of *C*. *albicans* hyphae. Following signal peptidase cleavage after amino acid 39 as previously predicted, HWP1 has a calculated Mr of 61,122. Staab et al. (1996).

The complete predicted open reading frame supports several inferences about the mechanisms leading to the expression of HWP1 on surfaces of *C. albicans.* The hydrophobic *N* and *C* termini are consistent with entry into the classical secretory pathway and modification with a GPI anchor (Englund, 62 ANNU. REV. BIOCHEM. 121-38 (1993)) respectively, as has been described for other yeast cell wall proteins in which GPI anchor addition precedes covalent linkage of proteins to the cell wall. Lu et al. 14(7) MOL. CELL. BIOL. 4825-33 (1994).

### II. Creation of mutant strains of C. albicans lacking HWP1, and a revertant that are otherwise isogenic with the parental strain.

Two genomic *Bam* HI fragments containing *HWP1* half-clones were isolated from a genomic library constructed in Lambda GEM-12 (Promega). Birse et al. The *Bam* HI fragments were cloned in pBluescript SK- to generate pGB8 (1.0 kb insert) and pGB23 (3.0 kb insert). An uninterrupted *HWP1* gene was created by ligating the 3.0 kb insert of pGB23 into pGB8 to create pGBHWP1. An inactive *HWP1* gene was created by replacing *HWP1* DNA between the *Bcl* I and *Bgl* II sites (394 bp) with the 4.0 kb *hisG-URA3-hisG* cassette of p5921. Fonzi & Irwin, 134 GENETICS 717-28 (1993). The new plasmid containing an interrupted *HWP1* was named pHWP1URA3 (Figure 1).

Both homologues of *HWP1* were disrupted (Staab et al. (1999)) using the "URA blaster" method adapted from the protocol developed for use with *S. cerevisiae* (Alani & Kleckner, 116 GENETICS 541-5 (1987)) described by Fonzi and Irwin. Fonzi & Irwin, 134 GENETICS 717-28 (1993). Plasmid HWP1URA3 was digested to completion with *Hind* III which cuts in the polylinker region and in the *HWP1* coding region to release a 5.3 kb fragment harboring the *hisG-URA3-hisG* disrupted *HWP1* gene. Five or 10 µg of digested pHWP1URA3 DNA was used to transform the ura strain CAI4 (Fonzi & Irwin, 134 GENETICS 717-28 (1993)). Postlethwait & Sundstrom, 177 J. BACTERIOL. 1772-79 (1995).

Transformants were screened for the desired recombination events by Southern blot analysis. Postlethwait & Sundstrom, 177 J. BACTERIOL. 1772-9 (1995). Of the twelve Ura⁺ transformants analyzed, three displayed the desired homologous recombination integration event at the *HWP1* locus. One strain, CAH7, was chosen for further manipulations. To remove the *URA3* selectable marker, *ura* auxotrophs of CAH7 were selected on media containing 5-fluoroorotic acid (Boeke et al., 197 MOL. GEN. GENET. 345-6 (1984); Fonzi & Irwin, 134 GENETICS 717-28 (1993)) prior to the next round of transformations to reestablish the Ura⁺ phenotype. All six Ura⁺ strains examined had the desired loss of *URA3* by homologous recombination between the *hisG* repeats. One strain, CAH7-1, was transformed with the disrupted *HWP1* cassette, and Ura⁺ isolates were screened for homologous integration of the transforming DNA at the intact *HWP1* locus. Two of nine transformants analyzed harbored the desired disrupted *HWP1* loci, and as expected did not have HWP1 on hyphal surfaces as detected by indirect immunofluorescence assay (IFA). Staab et al. (1996). An Ura⁻ auxotrophic derivative of CAH7-1 A was generated by selection on 5-fluoroorotic medium.

An *HWP1*+ revertant strain was created by using the *ura HWP1* strain, CAH7-1A1, as a recipient strain for equal amounts (5 µg each) of *Hin*dIII-digested pGBHWP1, and *Xba* I and *Xho* I-digested p24enura DNA. Postlethwait & Sundstrom, 177 J. BACTERIOL. 1772-9 (1995). Plasmid 24enura harbors an URA3-disrupted *C. albicans* enolase gene. Ura+ transformants were screened for *HWP1*+ phenotype by IFA of germ tubes, and one transformant, CAHR3, was further analyzed by Southern blotting. CAHR3 had the desired homologous recombination insertion at the *HWPI:: hisG* locus, and one of the enolase loci, was disrupted with *URA3.* There were no distinguishing phenotypic features between CAHR3 and CAH7-1 (Ura+ single disruptant). All of the strains produced germ tubes in liquid and in solid media except when dropped on the surface of agar media. Importantly, and as expected, the *HWP1*+ revertant strain (CAHR3) possessed HWP1 on hyphal and not yeast surfaces as detected by IFA. The immunofluorescent results show that not only was HWP1 gene replacement successful, but that the developmental hypha-specific regulation was also maintained. In other experiments, CAH7-1A was found to lack *HWP1* mRNA. Staab et al. (1999).

### III. Effect of HWP1 on adhesion of germ tubes to human buccal epithelial cells.

The isogenic *HWP1* strains created above were used to assess the function of HWP1 in adhesion to buccal epithelial cells. HWP1 allowed germ tubes to form stable attachments to BECs that were resistant to treatment with SDS and heat (stabilized adhesion). Stabilized adhesion was abrogated by known inhibitors of transglutaminases, iodoacetamide and monodansylcadaverine. The homozygous *HWP1* mutant strain, CAH7-1A, was unable to form stable attachments to BECs whereas the revertant strain and the heterozygous mutant, CAHR3 and CAH7, respectively, were unaffected in the ability to form stable attachments. In biochemical experiments, recombinant HWP1 was shown to be a substrate for mammalian transglutaminase and associate tightly with BECs envelopes after incubations under the adhesion assay conditions. Staab et al. (1999). The data points to the ability of HWP1 to mediate stable attachments to human tissue through interactions with host transglutaminases.

### IV. HWP1 is important for systemic candidiasis.

Mice (6/group) were injected intravenously with wild type or mutant strains of *C*. *albicans*. CAH7-1A, which lacks *HWP1*, was significantly reduced in the ability to cause lethal candidiasis compared to strains with *HWP1*.

### V. HWP1 is important for gastrointestinal candidiasis.

Beige nude, Epsilon 26 and beige het mice were raised in the Gnotobiotic Laboratory at the University of Wisconsin, Madison and were maintained under germfree conditions. Most mice were used at the age of 4 to 7 weeks, however a few pups and older animals were also included. Both male and female mice were used. Mice were monoassociated on Day 0 with *C. albicans* strains CAH7 (*HWP1*/*hwp1*), CAH7-1A (*hwp1*/*hwp1*), CAHR3 (revertant) and SC5314 (wild type) by swabbing the mouth with a suspension containing 10⁷blastoconidia per/ml. Mice were maintained in sterile isolators for up to seven weeks and watched for signs of ill health including lethargy, matted or thinning fur, chills, closed eyes, and emaciation that mandated sacrificing. Thus the terms "ill" and "survival" are synonymous and are used interchangeably in the description below. Fecal pellets were collected to verify that animals were colonized and to verify the identity of strains of *C*. *albicans* that the animals had received. Upon sacrificing, quantitative cultures of cecal contents verified the presence of *C. albicans* in individual animals. Quantitative kidney cultures were performed to determine whether *C. albicans* had translocated across the gastrointestinal tract.

Gnotobiotic beige nude mice that received *C*. *albicans* strains with *HWP1* were more prone to failure to thrive than mice that received the *C. albicans hwp1* null mutant strain (Figure 2). Only two of seven beige nude mice given the *HWP1*-containing heterozygous *C*. *albicans* strain and one of four mice given the *HWP1* revertant remained healthy for six weeks. In contrast, only two of 11 mice that received the *hwp1* null mutant strains of *C. albicans* became ill. The survival difference between groups of mice that received the *HWP1* heterozygote strain CAH7 compared to the *hwp1* null mutant was statistically significant ( P < 0.05). The combined groups of mice that received the *HWP1* containing heterozygous and revertant, also showed a significant difference in survival from mice receiving the *hwp1* null mutant strain. The absence of a significant difference in the survival of mice that received the revertant strain and the *hwp1* null mutant (P = 0.058) was because one of the mice was sacrificed for "comparison" prior to becoming ill. When this mouse is removed from the analysis, the survival difference between null and revertant is significant.

The survival of Epsilon 26 mice also depended on the presence of *HWP1* in *C. albicans* strains used for monoassociation. All mice that received *HWP1*-containing heterozygous *C. albicans* strains and four of five mice given the *HWP1* revertant strain became ill. Only one mouse that received the revertant strain remained healthy for seven weeks. In contrast, none of the five mice that received the *hwp1* null mutant strains of *C. albicans* became ill. The survival differences between groups of mice that received the *HWP1* heterozygote and revertant strains compared to the *hwp1* null mutant were statistically significant ( P < 0.01).

### Example 2: Regulation of the HWP1 gene

### I. Sequence of the 5' flanking region of HWP1.

The upstream region of *HWP1* that is expected to contain the promoter and regulatory sequences has been isolated as a 1.6 kbp *Bgl*II fragment of *C*. *albicans* genomic DNA. The cloned *HWP1* promoter region includes 1,467 bp upstream of the ATG start codon of the structural *HWP1* gene. Genetic organization of the upstream region is shown in Figure 3 with cloned DNA indicated in the shaded portion.

Features of the cloned DNA sequence are depicted in SEQ. ID. NO: 1 (Figure 4). A 10 nucleotide perfect direct repeat was found at positions 554 and 567 and a putative TATA box was found 67 bp upstream from the beginning of the transcript. Searches for potential binding sites in fungal matrices using MatInspector v2.2 revealed the presence of sequences for NIT2 (16 sites), PHO4 (2 sites), MATA1 (1 site), and HSF (1 site) binding sites within the 1,467 nucleotide sequence. A *S. cerevisiae* cAMP response element (CRE) site was also found within a PHO4 site. The presence of NIT2 and HSF sites are particularly interesting as there is evidence for nitrogen regulation and temperature regulation of germ tube formation in *C. albicans*

The predicted position for the TATA box is shown. A notable feature is a pair of NIT2 sites in opposite orientations near position 800 that form an almost perfect palindrome on opposite DNA strands.

Matches to UAS's or URS's were not found in the 613 bp figure between the left *Bgl*II site and the 5' neighboring gene's stop codon. *APL6* (homologue of the human AP-3 complex unit was mapped to this locus by the *C. albicans* Sequencing Project at Stanford University). These findings indicate that all of the necessary *cis* elements needed for developmental regulation are found within the 1,467 bp upstream of *HWP1.*

The numerous sites for NIT2 binding is significant because the NIT2 protein of *Neurospora crassa* is a global positive-acting transcription factor of nitrogen structural genes when preferred nitrogen sources are lacking. A MT2-like protein likely may regulate virulence genes in *C. albicans.*

A search of the *C. albicans* genome revealed that GAT99 (GAT-1 or GATA like protein) is highly homologous to NIT2. The GAT99 predicted amino acid sequence was also homologous to AREA, NRE and GLN-3. Referring to amino acid identity of GAT99 [SEQ. ID/ NO: 2] to the 50-residue DNA binding domain in NIT2 [SEQ. ID. NO: 3] was 92% by the ALIGN program and is shown in Figure 5. This region is highly conserved in GATA factors. Finding GAT99 in *C*. *albicans* suggests that regulation of *HWP1* expression may be tied to nitrogen regulatory circuits. In fungi, NIT2 sites in promoter regions vary in number, location, and orientation; strong NIT2 binding sites are usually found as two or more elements located within 30 bp of each other in the same or opposite orientation. There are three such pairs of NIT2 sites in the 1467 bp upstream region of *HWP1,* one set starting at bp 560, the second at bp 800 and the third at bp 1246. Since mutations in any of the nucleotides in the core sequence greatly reduce or eliminate NIT2 binding these NIT2 elements will be a focus of promoter mapping using deletion analysis and site-directed mutagenesis.

### II. Sequence of the 3' flanking region of HWP1.

1,441 nucleotides downstream of *HWP1* have also been cloned and sequenced. The end of transcription was determined by 3' RACE PCR using an *HWP1*-specific oligonucleotide corresponding to residues 691-710. The 3' DNA sequence of the RACE PCR product showed that translation of *HWP1* terminated with a UAA codon followed by 277 nucleotides of untranslatead message. A polyadenylation site (AAUAAA) 252 nucleotides from the stop codon, and a poly A tail of 14 adenosines were also part of the 3' region of the message. The polyadenylation site, and other sequences potentially important for 3' end formation are shown in bold.

Referrring to Figure 6 [SEQ. ID. NO: 4], a truncated open reading frame (bold-italics) consisting of 178 amino acids, beginning with an ATG and interrupted by the *Bam* H1 site at the end of the clone (underlined), exists at the 3' end of this region indicating the presence of an additional gene. The homologue *of RAD2* (excision repair gene) was mapped to this open reading frame by the *C. albicans* Sequencing Project at Stanford University. Studies of the open reading frame showed that this gene is not differentially regulated (mRNA is present in both yeast and hyphal forms).

### III. Kinetics of Expression of HWP1 mRNA.

Initial studies showed the presence of an abundant *HWP1* signal on Northern blots three hours after initiation of mass conversion from yeasts to hyphae (strain SC5314) in M199. To gain more information about the kinetics of expression of *HWP1*, Northern blots were performed on cells placed in prewarmed M199, Lee's pH 6.5 medium, and M 199 with 5% fetal calf serum beginning at 10 minutes and continuing for 7 hours (Figure 7). Under these conditions, close to 100% of the cells form germ tubes; no budding yeasts were observable. Each lane contains 2.5 ug of total RNA. The presence of nearly equivalent amounts of RNA in all lanes was verified by using an18S ribosomal RNA probe which controls for sample amounts in lanes. Oligonucleotide primers representing nucleotides 314 to 332 and complementary to 967 to 986 of 18S rRNA gene were used to amplify a 672 bp fragment by PCR. The PCR product was radiolabeled and used to probe stripped Northern blots. The blots were first probed with *HWP1* cDNA which represents the 5' region of the structural gene that encodes the proline and glutamine-rich repetitive N-terminal domain HWP1.

Referring to Figure 7, the blot has been overexposed to show that *HWP1* mRNA is detectable within 20 minutes and at 7 hrs of placing cells under induction conditions (*HWP1* mRNA is clearly visible in the autoradiograph but is faint in the reproduction above). The message level increases dramatically between 20 and 30 minutes and continues to rise, peaking at 3-4 hr. After four hours, the *HWP1* mRNA begins to decline until 7 hr, where it stabilizes at a low level.

To correlate the presence of *HWP1* mRNA with protein (HWP1) and germ tube formation, indirect immunofluorescence experiments were performed using monospecific antiserum to recombinant HWP1 (rHWP1). Germ tube production and surface HWP1 production are synchronized, both appearing at 45 min on approximately 25% of yeasts and are seen on the majority of cells by 60 min (not shown). Messenger RNA precedes the presence of visible germ tubes and surface HWP1 by 20-25 minutes.

The kinetics of *HWP1* expression appears to differ quantitatively from mRNA for house keeping genes, but the overall rise and fall of message levels is similar. *HWP1* message is evident nearly an hour earlier than *ENO* mRNA, a gene which encodes an abundant glycolytic enzyme, enolase, that is present in yeast and hyphal growth. However, both mRNA's peak at 3-4 hours and drop thereafter. Similar results were found for other non-developmentally regulated gene, *ACT1* and carboxypeptidase Y. These results show that expression of *HWP1* is produced earlier and to higher levels than housekeeping genes, reflecting the presence of activation mechanisms not found for housekeeping genes or at least in addition to those for housekeeping genes.

The results show that repressive mechanisms serve to shut off *HWP1* during yeast growth, through interactions of *HWP1* upstream sequences and DNA-binding proteins that interact with *TUP1* or other general repressor proteins. The results also show that activating factors may serve to increase *HWP1* mRNA levels above those for other abundant proteins such as enolase and actin during germ tube growth.

### Example 3: Creation of an HWP1:GFP reporter fusion for monitoring the HWP1 promoter

*HWP1*:GFP reporter fusion was created for monitoring the *HWP1* promoter and for verifying that the appearance of GFP accurately reflects the activity of the native *HWP1* promoter.

The strategy was to target a transcriptional fusion between *HWP1* flanking regions and a reporter gene (GFP) to the genomic enolase locus. The reason for inserting the reporter into the genome is to avoid artifacts associated with overexpression and problems with plasmid instability that are inherent in the use of *C. albicans* ARS (CARS) plasmids. Unfortunately, plasmids with features such as *S. cerevisiae* CEN plasmids which have centromeric sequences that confer stability and low copy number are not available for *C. albicans*.

The choice to use the *ENO* genomic locus was made for several reasons, the first being convenience. A construct (p24enura) having the *C. albicans* enolase gene disrupted with *URA3* (58) was used. A second reason is that the effect of gene disruption at the *ENO* locus of *C*. *albicans* is known. Preliminary work has shown that disruption of one of the *C*. *albicans* enolase genes does not adversely affect growth, morphogenesis (Postlethwait & Sundstrom, 177 J. BACTERIOL. 1772-9 (1995); Staab et al. (1999)) or pathogenesis in a murine model of systemic candidiasis. The virulent *HWP1* revertant in the systemic and mucosal candidiasis studies was prepared using co-transformation with p24enura. Staab et al. (1999). Studies with UnoPP-1 ((Postlethwait & Sundstrom, 177 J. BACTERIOL. 1772-9 (1995)) (and the *HWP1* revertant (Staab et al. (1999)) showed that enolase gene disruption also does not affect the frequency of germ tube formation, the length of germ tubes that are formed or expression of HWP1 on germ tube surfaces.

In principle, use of the *ENO* locus is similar to use of the *ADE2* locus for studies of promoters of genes involved in switching (Srikantha et al.) and, thus, a precedent has been set for studying promoter activity at ectopic loci. *ADE2* and *ENO* are both constitutively expressed, housekeeping genes. There is no known reason why the *ENO* locus would be more cumbersome to work with than *ADE2.* The availability of the *ADE2* locus does serve as a fallback gene should problems with the *ENO* locus arise. The *ADE2* gene is cloned and could new constructs utilizing *ADE2* must be made.

The *HWP1* locus is not particularly useful for this work because it is necessary that promoter sequences that are manipulated and introduced into the genome are studied. If the construct were introduced at the *HWP1* locus, native promoter sequences would probably confound the results. The experiments depend on being able to delete and add back portions of the region upstream of the promoter. This requires use of an independently replicating, stable plasmid which is not available for *C. albicans,* or ectopic expression.

### I. Plasmid construction.

To be able to easily assess the role of *HWP1* flanking sequences in gene expression, the coding region of *HWP1* was replaced with that of the green fluorescent protein (GFP) which has been optimized for expression in *C. albicans* by Cormack. Cormack et al. The transformation recipient is the ura auxotroph CAI4. The presence of a functional *HWP1* gene in this strain are used to correlate the expression of GFP message with *HWP1* message, confirming the utility of the reporter strain as accurately reporting *HWP1* expression.

The features of the plasmid that was constructed according to the scheme in Figure 8 are:
1) 1,467 bp of *HWP1* upstream flanking sequences;
2) 352 bp of downstream flanking sequences;
3) the *C*. *albicans* enolase gene disrupted with the *C. albicans URA3* gene for selection in the *ura3* strain CAI4, and for targeting to of one of the *ENO* gene loci of *C. albicans;* and
4) a plasmid lacking *HWP1* upstream sequences is used as a negative control.

### II. Transformation of C. albicans and comparison of GFP expression to expression of the native chromosomal HWP1 gene.

Plasmid *HWP1*GFP1 was digested with *Cla* I (arrow; see Figure 9) which cuts at a single site in the *C. albicans* enolase gene to target the construct to the ENO locus in the genome using spheroplast transformation. This resulted in a mutated copy of the enolase gene disrupted with *URA3* adjacent to a wild type copy as diagrammed below. Transformants were selected on media lacking uridine. A control plasmid lacking *HWP1* upstream regions but otherwise identical to pHWP1GFP1 (pGFP1) served as a negative control.

Transformants were first screened by Southern blotting to verify integration at the enolase locus. The Southern blotting experiments were also used to determine the copy number of pHWP1GFP1 DNA inserted into the genome, since it is possible that more than one copy of DNA will become inserted. The copy number of inserted plasmids were determined by comparing hybridization intensities of DNA from transformants with a standard curve of GFP DNA to identify transformants with a single copy of GFP DNA. This procedure was applied in all the following experiments to ensure that strains with single integrations will be compared for GFP levels.

GFP expression under the control of the *HWP1* promoter had been achieved. GFP was detectable in cells making germ tubes and was not detectable in yeast form cells. The result showed that the *HWP1* flanking sequences present in the plasmid not only permit expression of GFP, but that they also confer hypha-specific regulation to GFP, confirming that the sequences in the plasmid do have controlling elements. Photomicrographs showing GFP expression in germ tube forming cells that were taken with an epifluorescence microscope and light photomicrographs of the same field showing that only cells with germ tubes fluoresced.

An important point that was elucidated by these unstable constructs is that position effects are not likely to play a major role in expression of *HWP*1*.* Since integration into the chromosome was not achieved, developmental control was mediated solely by plasmid sequences. Additional evidence for the lack of a role for position effects is the absence of developmental regulation of the *RAD2* gene which is just downstream of the *HWP1*

Stable integration of the *HWP1* promoter: GFP construct at the genomic enolase locus have also been achieved. Linearization of the construct at the *Cla* I site in the enolase coding region followed by transformation of CAI4 with 10 µg of DNA produced four transformants. Two of these (GFP1/C2 and GFP1/C3) were stable, retaining the plasmid in the absence of selection. Southern blotting of genomic DNA digested with *Ava*I confirmed that both transformants had integrated GFP into the ENO locus of *C. albicans* (Figure 10). The integrated construct produced a 7.8 kb *Ava*I band that was recognized by both enolase and GFP probes. The undisrupted enolase locus produces a 6.4 kb band (Figure 10, very faint band in lane 4, see arrow). As expected, GFP sequences are not found in the parental strain, CAI4 (Figure 10, lane 6).

Several growth conditions were employed to show that the *HWP1* promoter:GFP construct is developmentally regulated. GFP1/C2 and GFP1/C3 transformants were grown to stationary phase in YNB (yeast nitrogen base with 50 mM glucose) for 48 hrs at room temperature before placement in prewarmed (37°) M199, fresh YNB at 27°, or in the four Lee's media conditions (pH 4.5 and 6.8 at 27° and 37°) with gentle shaking at the appropriate temperature for 3 hrs. The cells were examined for the production of GFP in germ tubes incubated in M199 and Lee's pH 6.8 at 37°. As expected, fluorescence was not localized to germ tubes but was found throughout the cytoplasm of *C. albicans* cells because GFP does not have a signal sequence or other features that mediate secretion and cell wall expression that are present in *HWP1.* In addition, the appearance of fluorescence coincided with the induction of germ tube formation (Figure 11, Panel A). Yeast (Figure 11, Panel B) and pseudohyphae (Figure 11, Panel C) forms did not express GFP.

Given the lack of GFP expression in yeast forms, it is unlikely that the GFP construct will fluoresce without the flanking sequences. However, as an absolute control for background fluorescence, a plasmid lacking upstream sequences is prepared. Fluorescence in expressing strains are compared to strains transformed with the GFP-ENO-URA3 portion of the plasmid localized to the ENO locus. DIC differential interference microscopy and the ability to merge DIC images with fluorescent images are essential for these experiments.

To show that GFP does not interfere with *HWP1* expression, indirect immunofluorescence assays were performed to detect surface HWP1 using GFP-producing transformants (Figure 12). Secondary anti-rabbit antibodies conjugated with Texas Red rather than FITC were used, so that HWP1 could be detected in the presence of GFP. No differences were noted in the appearance of HWP1 on GFP-producing cells compared to wild type cells and parent strains.

The lack of inhibition of *HWP1* expression by the GFP construct is fundamentally important in that it shows that artifactual influences on mechanisms regulating *HWP1* expression will not occur.

Fluorescence microscopy will allow rapid, qualitative assessment of GFP production but will not permit quantitative measurements. Flow cytometry is a straightforward and simple method to determine fluorescence levels. The number of cells and their relative fluorescence are determined by flow cytometry after analysis of 10,000 events. The amount of fluorescence for a defined number of individual cells (the actual number of cells necessary will be determined in preliminary experiments) is quantified using an image analysis system. An example of flow cytometry data used to analyze the stable transformants is shown in Figure 13. The graph in Figure 13 (Panel B) shows a three log shift in fluorescence of germ tubes; cells (yeasts) that did not germinate (approximately 20% of the cells) are non-fluorescent (Figure 13, Panel A).

Fluorometry using glass bead cell extracts and a fluorometer is also be used to quantitate GFP fluorescence. The emission at 505 nm is read with excitation at 488 nm. The advantage of fluorometry is the ability to measure fluorescence for a given amount of protein extract which permits detection of subtle changes in fluorescence that may not be apparent by flow cytometry. Both methods are routinely used to monitor GFP expression and the results from the two methods are expected to be comparable.

To further ensure that GFP expression reflects *HWP1* expression, the kinetics of production of GFP mRNA was correlated with the kinetics of expression of *HWP1* mRNA. *HWP1* mRNA appears within 20 minutes of placing yeasts in germ tube induction conditions. Northern blotting with the reporter strain is also performed, and GFP mRNA is detected by hybridization and correlated with the presence of *HWP1* mRNA. M199 is used at 37°C without serum in these experiments the kinetics of induction are independent of the composition of the media. GFP fluorescence is expected to coincide with the appearance of *HWP1* mRNA and precede the emergence of germ tubes.

The kinetics of the disappearance of GFP mRNA is correlated with the disappearance of*HWP1* mRNA when cells are transferred from induction to repressing conditions. Two methods for repression are employed. In the first method, cultures of 100% germ tubes that have been growing as germ tubes for 2.5 hours are rapidly subjected to a 10-fold reduction in culture volume by filtration (*i*.*e*., a 1 liter culture will be reduced to 100 ml by filtration). A second method involves dropping the temperature by filtering the 2.5-hour germ tube culture and resuspension in M199 at 30°C. In both cases, transferred organisms in repressing conditions are removed at 5 min intervals and mRNA is isolated followed by Northern blotting using probes for *HWP1* and GFP mRNA. The morphology of the cells is monitored by microscopy and the appearance of cells is documented by photomicroscopy using a CCD camera. Thus the kinetics of GFP expression is correlated with *HWP1* mRNA expression during induction and repression of germ tube formation.

### Example 4: Identification of putative UAS and URS elements that regulate HWP1 expression.

### I. Deletion analyses.

The locations of *cis*-acting regulatory sequences upstream of *HWP1* is determined by performing the reporter experiments using plasmids which contain deletions in *HWP1* upstream sequences. The location of UAS's is detected by the correlating the loss of GFP expression with the deleted region. Constructs lacking 5' sequences are also be assessed.

The initial analysis of the *HWP1* promoter region is to identify regions that affect expression by deletion analysis. DNA fragments having less and less 5' DNA but the same 3' sequence for fusion to the open reading frame of GFP were created (Figure 14). Sequential deletions of DNA are be performed by PCR with oligonucleotides to sequences progressively closer to the -1 site [SEQ. ID. NOS: 5-11]. The same 3' oligo (Figure 14) used to generate the original plasmid construct (pHWP1GFP1) are used. The same plasmid (pBSBglIIl.8) with 5' *HWP1* genomic DNA is used as template for PCR. DNA sequences specifying a *Hind* III site on the 3' oligonucleotide *and Xho* I sites on the 5' oligonucleotides resulted in the addition of Hind III and Xho I sites on the 3' and 5' ends of the PCR products, respectively.

The strategy described above for constructing deletions required the removal of the Hind III site in enolase by site-directed mutagenesis using the Promega GeneEditor *in vitro* Site-Directed Mutagenesis System (Madison, WI). This high efficiency system allowed for the generation and selection of oligonucleotide-directed mutations. The method takes advantage of an *E. coli* strain mutated at the MutS gene (DNA repair) which permits recovery of plasmid DNA without selection against the desired mutation. The system also allows for generating simultaneous mutations using one template, a feature that will become useful to study the effects of various mutations in a single construct on GFP transcription.

A mutagenic oligonucleotide that mutates the HindIII site in enolase is generated. The loss of one HindIII site is used to screen potential mutants, and the desired base change in enolase is verified by DNA sequencing. The resulting plasmid is named pHWP1GFP2, and is used as pHWP1GFP1 in transformations of *C. albicans.* The loss of the *Hind*III site in enolase should not affect the developmental regulation of GFP observed with the original pHWP1GFP1 construct since enolase sequences are being used to target integration in the chromosome and are not driving GFP transcription. Before the PCR products are cloned into pHWP1GFP2, the original 1.46 kb 5' *HWP1* fragment is separated from the rest of the construct by gel electrophoresis. The gel-purified construct minus the 1.46 kb fragment is then be used as the vector for cloning the various PCR-generated deletions.

The linearized plasmid with XhoI and HindIII ends is also used to generate a promoterless construct. The 5' overhang ends produced by XhoI and HindIII is blunted by using Klenow DNA polymerase fragment in the presence of dNTPs. Self-ligation of the blunt-ended plasmid produces a construct without a promoter that is named pGFP1. The oligonucleotides for generating the first set of deletions are shown in Figure 14 [SEQ. ID. NOS: 5-11].

Growth Conditions: To identify UAS's, and URS's three independent, single copy, transformants for each construct are analyzed in conditions that induce the appropriate form of growth. The conditions are:
For germ tubes;

| | |
|---|---|
| 1. | M199 at 37°C* |
| 2. | M199 with 5% serum at 37°C* |
| 3. | Lee's medium at 37C, pH 6.8* |

| | |
|---|---|
| *Clarified, whole human saliva at 37°C | |

For yeasts;

| | |
|---|---|
| 1. | YNB at 37°C^{*} |
| 2. | Lee's media, 37C, pH 4.5^{*} |
| 3. | YNB at 25°C ^{*} |

| | |
|---|---|
| *Both liquid cultures and agar plate media will be analyzed. | |

UAS's important for *HWP1* expression are identified by a decrease in expression of GFP during germ tube inducing conditions compared to the strains with the intact *HWP1* promoter region. URS's important for repression of *HWP1* are identified by *de novo* expression of GFP during yeast growth. Liquid cultures are analyzed by flow cytometry and fluorometry. For fluorometry, GFP fluorescence in extracts is determined relative to the amount of protein in extracts. The average values for three transformants is used. A given deletion is considered to harbor a UAS element if its deletion leads to at least 30% reduction in GFP levels compared to fluorescence of transformants with the intact construct, and to harbor a URS element if its deletion leads to at least a 3-fold enhanced expression during growth as yeasts. Fluorescence of solid cultures is assessed qualitatively using the fluorescence microscope.

### II. Fine structure mapping and site-directed mutagenesis of DNA segments that are predicted to be functionally important.

Deletion analysis provides strong preliminary information about the location of regulatory elements within approximately 200 bp. This section performs fine structure mapping of the regions deemed important, as set forth in section I. For example, if the experiments note a loss in regulatory elements in the 200 bp segment upstream of the TATA element, then the specific location of the regulatory element is determined by the combined results of several approaches:
1). The smallest construct containing the elements is analyzed by deletion analyses similar to those described above, but by constructing a new deletion series within the region of question to generate a series of overlapping deletions differing by 50 bp in size.
2). Site-directed mutagenesis using the Promega GeneEditor *in vitro* Site-Directed Mutagenesis System is used for generation and selection of oligonucleotide-directed mutations within the 200 base pair region. The intact *HWP1* promoter region:GFP construct is used as the template for mutagenesis and the exact sequences to be mutated will depend on the actual sequence and the results of the fine structure deletion mapping above.

### III. Impact of regulatory elements on minimal promoter.

The most deleted HWP:GFP construct retains the TATA box and is predicted to bind basal transcription factors. Addition of regulatory elements to this putative "minimal promoter" may lead to an increase or decrease in basal promoter activity. The effect of DNA with UAS or URS elements on this highly deleted construct is more easily predicted once the minimal GFP fluorescence from this construct is determined.

### Example 5: Characterization of DNA binding proteins (DNABP) that regulate the HWP1 promoter.

### I. Electrophoretic mobility shift experiments.

*cis*-acting regulatory sequences that are the most potent regulators of GFP expression are used to identify and characterize binding proteins using electrophoretic gel mobility assay (EMSA) experiments. Protein extracts containing the putative DNA binding factors are prepared from germ tubes and/or yeasts grown under conditions where regulatory activity is present based on the experiments using the media described above. Protein extracts are pelleted as ammonium sulfate precipitates. Covitz & Mitchell, 7(8) GENES DEV. 1598-608 (1993). Cells are washed, lysed with glass beads in buffer with protease inhibitors, clarified by low speed centrifugation followed by ultracentrifugation to remove insoluble material. DNA is removed from the protein extracts by precipitation with polyethyleneimine followed by centrifugation. Proteins in the supernatant are precipitated with solid ammonium sulfate, followed by collection of the pellet by centrifugation. Extracts from hyphal and yeast growth forms are prepared using multiple media conditions described below.

The EMSA method of Chodosh is used. Chodosh, CURRENT PROTOCOLS IN MOLECULAR BIOLOGY (Ausubel et al., eds.) (1988). Fragments exhibiting the strongest UAS and URS activities, as determined above, are used as probes. Double-stranded oligonucleotides are prepared from these regions and are end labeled with [γ-³²]ATP. Extracts and probes are incubated in binding buffer containing glycerol, and poly(dI:dC) prior to separation by electrophoresis with a high-ionic strength 4% polyacrylamide gel. Gels are dried and examined by autoradiography.

The sizes of retarded complexes are compared to the mobility of the labeled DNA fragment in the absence of added proteins. The number of complexes, and their correlation with yeast or hyphal morphology is assessed. To verify the specificity of the complexes, cold probe present in 50 fold excess is added as a competitor in the incubations. If the shifted bands are specific for the labeled probe, the bands should disappear in the presence of nonradioactive competitor probes.

The gel mobility shift experiments provide insight into the regulation of *HWP1* expression. There may be proteins in DNA-protein complexes that are specifically present when hyphal-extracts but not yeast extracts are used. If repression during yeast growth is important, yeast-specific complexes will be found. Alternatively, there may be complexes that are present when extracts from either yeasts or hyphae are used, or there may be both morphology-specific as well as non-specific complexes. Growth phase-specific complexes if present will be the focus of future studies. However, the absence of growth phase-specific complexes is also of interest since hypha-specific expression might be a result of differential activation of upstream binding proteins through protein modification such as phosphorylation, or there might be additional protein-protein interactions that determine specificity.

### II. Determining sizes of DNA binding proteins.

Determining the size of the protein(s) that form complexes with *HWP1* upstream DNA is necessary for subsequent efforts to clone the corresponding gene(s) from expression libraries. To determine the sizes of proteins complexed to *HWP1* upstream DNA, complexes are crosslinked using UV light, followed by elution from the non-denaturing gel, solubilization in Laemmli-SDS sample buffer and separation on SDS-PAGE gels, followed by autoradiography as previously described by Chodosh. The sizes of the proteins are determined using a standard curve of proteins of known molecular weights.

### III. Cloning genes encoding DNA binding proteins.

After the detection and preliminary characterization of proteins that bind to the upstream region of *HWP1*, the genes for the binding protein of interest are cloned. Having the primary sequence for proteins that bind to the upstream promoter region of *HWP1* provides information for future investigations into the mechanisms of morphology-specific gene-expression of *HWP1.*

DNA affinity chromatography is the method of choice for isolation of DNABPs. The UAS and URS elements identified in previous sections are used to isolate the desired regulatory factors in native form from nuclear extracts of *C. albicans* cells. Primers that bind to DNA in the *HWP1* promoter region template that surround putative UAS and URS elements of interest are amplified by PCR to generate products 80-100 nucleotides in size. Primers for PCR reactions are synthesized with a 5' biotin group. PCR products are separated on and excised from agarose gels. The biotinylated PCR products are then immobilized on Streptavidin-conjugated Dynabeads as described by Gabrielsen and Huet (Gabrielsen & Huet, 218 METHODS ENZYMOL. 508-25 (1993)) followed by washing to remove unbound DNA.

To purify DNABPs, nuclear extracts from cells grown in appropriate media are incubated with the DNA-beads in binding buffer containing poly (dI-dC) and washed, followed by elution of the DNA-binding protein with high salt, and then desalting. Myokai et al., 96(8) PROC. NATL. ACAD. SCI. USA 4518-23 (1999). To obtain a nucleic acid probe for screening cDNA and genomic libraries, purified DNA-binding proteins are subjected to N-terminal amino acid sequencing and/or sequencing of peptide fragments that were purified by HPLC.

Degenerate DNA sequences derived from the amino acid sequences are used to search the *C. albicans* genome which is now complete to 5X coverage. Once the DNABPG is identified by the initial search, PCR primers representative of the exact sequence in the genome are designed and used to amplify the gene using genomic DNA as a template. A possible problem is that the degenerate sequence may or may not successfully locate the corresponding genomic DNA in a computer search of the genome depending on the degree of degeneracy. If available, the sequenced peptides of the isolated DNABPs are readily identified and this will point to the exact DNA sequences needed for primer design. If the proteome is not available, and if the degenerate DNA sequences do not successfully locate the DNABPG, other methods will be used to isolate the corresponding gene. Degenerate oligonucleotide primers are designed to use in PCR to amplify a region of the DNABPG using genomic DNA as template to generate a probe for library screening. The DNA sequence of the PCR product is analyzed to verify the authenticity of the product as encoding the protein fragment. Library screening follows standard techniques that have been used to isolate genomic clones of other genes by those skilled in the art. Subclones in pBluescript® of large lambda clones are obtained followed by DNA sequencing to verify the identity of the cloned DNA as being representative of the PCR product and peptide sequences derived from the DNABP.

### IV. cDNA library screening using UAS or URS elements as probes.

An alternative approach is to screen a *C. albicans* cDNA expression library with the *HWP1* upstream sequences identified in the reporter gene experiments and gel mobility shift experiments described above. Currently-available cDNA libraries consist of cDNA prepared from mRNA isolated from germ tubes grown in M199. A yeast cDNA library is prepared, if necessary as determined by experiments or delineated above, to clone genes for proteins that bind to URS's using mRNA from yeasts in logarithmic growth. Because lambda plaques generally contain small amounts of fusion proteins in native conformation, the screening sensitivity is enhanced by concatenating the probe so as to generate DNA fragments with multiple copies of the binding sites. Plaques that hybridize with the probe (critical *cis*-acting sequences from previous experiments), but not with a control DNA probe are purified, and inserts are sequenced and analyzed for open reading frames.

To verify that genomic clones encode DNABPs of the expected activities, recombinant DNABPs is produced in *E. coli.* Very few *C. albicans* genes have introns, but if introns are present (as detected by the presence of conserved splice sites and stop codons within the coding region) cDNA clones are obtained by screening the appropriate cDNA library prior to production of recombinant proteins. The cDNA clones are used for production of recombinant proteins in *E. coli* as is routinely done by those skilled in the art using either pGEX or his-tag expression systems, and purified by affinity chromatography. If production of recombinant proteins in *E. coli* is not possible due to codon bias, the yeast *Pichia pastoris* (Invitrogen) will be used. Various recombinant HWP1 proteins expressed in *P. pastoris,* and vectors that fuse "his" tags to recombinant proteins are now available. The recombinant proteins are assayed by gel mobility shift experiments using the previously identified *cis*-acting sequences as probes, as well as SDS-PAGE analysis to compare the size of the purified protein to that initially found in crude extracts, procedures that provides confidence that the correct proteins that bind *C. albicans* UAS and/or URS sequences are being studied. Purity is assessed by the demonstration of a single spot on two dimensional electrophoresis.

### V. Characterization of DNA binding proteins by computer analysis of the predicted open reading frame.

cDNA clones isolated from library screening are used to screen a genomic library of *C. albicans.* Genomic clones containing proteins that bind regulatory sequences of *HWP1* are completely sequenced on both strands. The BLAST program is used to search the database through the National Center for Biotechnology Information to identify homologues in other organisms. The predicted protein sequences are extensively analyzed for the presence of hydrophobic segments, clustering of acidic and basic residues, tyrosines and phenylalanines, protein kinase target sites, nuclear localization, zinc finger, helix-turn-helix, homeodomain motifs and leucine zippers that are known to exist in other DNABPs, as well as for the presence of other amino acid motifs that specify functional attributes of proteins as well as for secondary structure.

### IV. Localization of DNA binding proteins.

DNABPs exert their effects after localizing to the nucleus. The presence of protein in the nucleus should correlate with the growth stage-specific binding determined in the gel mobility shift assays. To monitor expression of the DNABP in experiments described below, DNA encoding a c-myc tag that has been engineered to reflect the codon usage of *C. albicans* is fused to the recombinant protein. The tag is fused to the C terminus of the protein unless suspicions arise that predict important functions for the C terminus, or that predict that the C terminus is unexposed in the interior of the protein. In these cases, secondary structure predictions and predictions about conserved functional motifs are considered in positioning the c-myc tag in a location that is exposed on the surface of the protein for interaction with commercial anti-c-myc monoclonal antibodies.

The c-myc-tagged DNABPG is introduced into CAI4 by spheroplast co-transformation at its native locus. Co-transformation protocols targeting enolase disrupted with *URA3* to the enolase locus may serve as selection for DNA uptake. Integration of the c-myc-tagged DNABPG at its chromosomal locus is performed by Southern blotting. A potential problem with the previous approach is that creation of the c-myc-tagged DNA might cause artifactual localization. Therefore analysis of the native protein may be required. Monoclonal antibodies to purified recombinant DNABPs are also be prepared, to analyze native, untagged proteins.

Additionally, the monoclonal antibodies are also used in super-shifting of bands in EMSA assays to verify that the cloned gene represents the protein in extracts that cause only mobility shifts.

For localization experiments, yeasts and/or hyphae are formalin-fixed, followed by digestion of cell walls with Zymolyase and application to poly L-lysine coated microscope slides according to standard techniques. Pringle et al., 194 GUIDE TO YEAST GENETICS AND MOLECULAR BIOLOGY 565-601 (Guthrie and Fink, ed.) (1991). Cell membranes are permeabilized with methanol and acetone and cells are then be incubated with antiserum prepared to purified recombinant proteins, or commercial monoclonal antibodies to c-myc followed by affinity purified FITC-conjugated goat anti-mouse antibodies. As a positive control for nuclear localization, control cells are stained with DAPI, which binds to DNA. Negative controls consist of antibodies to enolase, a cytoplasmic protein which should not give nuclear staining and rhodamine-conjugated phalloidin which stains actin and also does not stain nuclei. These experiments verify that the DNABPs are localized in the nucleus and are present in the appropriate growth form.

### V. Role of DNA-binding proteins in expression of HWP1.

To determine the function of the DNABPs in expression of *HWP1* as well as in the bud/hyphae transition, gene disruptions will be performed. The disruptions are performed using the standard URA-blaster protocol for *C. albicans* (Fonzi & Irwin, 134 GENETICS 717-28 (1993)), which was used to disrupt *HWP1.* To ensure that the resulting phenotypes are truly associated with loss of the DNABP, revertant strains that have the DNA-binding protein gene replaced are also prepared. Co-transformation was used to obtain *HWP1* revertants in double knockout strains, and this approach is used to obtain revertants for these studies as well. If co-transformation is unsuccessful, constructs with the DNA-binding protein gene adjacent to the selectable *URA3* marker are prepared, which are used to transform ura- double knockout strains lacking the DNABPG.

The role of the DNA-binding proteins in *HWP1* expression is determined using conditions during which HWP1 is normally present. *HWP1* expression in solid media is also assessed, since morphogenesis in non-liquid environments may be relevant to disease and because germ tube formation is sometimes enhanced when organisms are within or on top of agar or other non-liquid media. Anderson et al., 52(2) INFECT. IMMUN. 458-67 (1989). *HWP1* can be detected on organisms excised from agar surfaces. For germ tube formation in solid media, yeasts are added to agar media (M199 or Lee's media at neutral pH) at 45°C just prior to pouring as set forth in example 2. Plates are incubated overnight at 30°C, allowing pinpoint yeast colonies to form within the agar. Plates are then be transferred to 37°C for induction of germ tube formation. The extent of germ tube formation is visualized and photographed using a stereomicroscope equipped with an automatic camera.

### VI. Role of DNA-binding proteins in adhesion of C. albicans to human buccal epithelial cells.

Null mutants for genes that activate *HWP1* expression are compared to isogenic strains with the gene in adhesion assays to determine if the loss of the DNABPG affects adhesion. If *HWP1* is not expressed, no stabilized adhesion is expected, since *HWP1* is the only gene encoding a transglutaminase substrate in *C. albicans*. It is possible that conventional adhesins may be co-regulated with *HWP1* and that the expression of the conventional adhesins may be absent in the null mutant. Both stabilized and conventional adhesion assays are performed as previously described. Staab et al. (1999).

### Example 6: Identify global regulatory networks by isolation of genes that are co-regulated with HWP1 using mini-array technology.

Global regulatory circuits that lead to the production of hypha-specific genes are also identified. Many examples in fungi exist where global regulatory circuits affect multiple genes that are necessary for growth in specific environments. It is likely that *C. albicans* employs such a regulatory circuit for expressing multiple genes during hypha-specific growth conditions. The DNABPGs identified provide a valuable tool for probing the *C*. *albicans* genome to identify global regulators.

### I. Role of DNA binding proteins in controlling expression of known hypha-specific genes.

Several genes other than *HWP1* have been reported to be hypha-specific. These are *HYR1, ECE1, ALS3, CHS2,* and *SAP6.* Bailey et al.; Birse et al.; Gow et al., 91(13) PROC. NATL. ACAD. SCI. USA 6216-20 (1994); Hoyer et al.; White & Agabian, 177(18) J. BACTERIOL. 5215-21 (1995). To determine if the DNABPG acts exclusively on *HWP1* upstream DNA, or if other hypha specific genes are subject to the same regulatory effects, Northern blotting is performed using RNA from isogenic strains that have or do not have the DNABPG(s). Equal amounts of RNA from the various isogenic strains that were grown in germ tube-inducing conditions is loaded on gels, and probes for ribosomal RNA are used as internal controls for equivalent loading. Probes, radiolabeled with [α-³²P] deoxycytidine by the random primer method, include the coding regions of the *HWP1* gene, the *HYR1* gene, and other hypha specific genes. If the DNABPG is important for expression of a given hypha-specific gene, either as an activator or as a repressor, the level of mRNA for the hypha-specific gene is decreased or increased respectively in the null DNABPG mutant compared to isogenic strains (including a complemented strain) harboring the DNABPG. The relative intensities of signals on the membranes is determined by analysis on a PhosphorImager.

The upstream regions of known hypha-specific genes is also be analyzed for the presence of the UAS and URS elements that were found to be important. The presence of these elements and the Northern blot results using RNA from strains having the DNABPGs are compared to determine the relationship between gene expression and presence of the regulatory elements. The data generated from these studies help determine the relationship between hypha-specific gene expression and the presence of putative DNABPGs.

### II. Regulation study of the DNABPGs.

It is possible that the DNABPG's isolated are produced in response to a factor that mediates the transcription of the identified genes, and that other layers of regulation are involved in expression of hypha-specific genes. If a developmentally DNABPG is identified then studies similar to the ones performed on the *HWP1* upstream region is performed on the upstream region of the DNABPBs. A reporter construct under the control of the new promoter region is constructed. UAS and URS elements in this DNA sequence are identified with the goal of finding upstream effectors in the networks that regulate hypha-specific gene expression.

### III. Mini-array-based, transcriptional analysis of a C. albicans genomic library to identify and characterize genes under the control of DNA binding proteins.

One may identify unknown target genes that are under the control of the DNABPGs isolated above. In the near future, this type of experiment will be performed using purchased chips or membranes spotted in known locations with oligonucleotides or PCR products representative of each open reading frame in the genome. Unfortunately, *C. albicans* chips and membranes are not yet available; however, a "work-around" method using primers that bind to standard *E. coli* vectors is proposed (*see* Figure 15).

The proposed method is comparable to differential screening of a genomic library with cDNA (prepared from mRNA) probes obtained from cells growing under differing conditions. This new proposed method is predicted to be more sensitive than differential screening, and the extensive plaque purification and subcloning steps inherent in standard library screening protocols is not employed. The method is being used to study *H*. *capsulatum* genes and was originally proposed by J. DeRisi who has extensive experience with genome wide expression monitoring. DeRisi & Iyer 11 (1) CURR. OPIN. ONCOL. 76-9 (1999). The method is diagrammed in Figure 15.

The basic approach is to use RNA from the isogenic strains with or without the DNABPGs that were grown in germ tube-inducing conditions to prepare labeled cDNA. PolyA+ RNA are prepared from total RNA using an oligo dT column and are used to prepare ³³P-labeled first strand cDNA to probe membranes spotted with *C. albicans* genomic fragments prepared as described below.

The mini-array approach using PCR products spotted onto nylon membranes follows procedures described for *S. cerevisiae.* Cox et al. The use of nylon membranes rather than glass chips is chosen because of the greater ease of sample application and because of the ability to analyze the results using ImageQuant software and a PhosphorImager.

The membranes are prepared by robotically spotting PCR products amplified from genomic inserts, 0.5-2.0 kb in size, cloned in pBluescript using universal primers (see Figure 14, arrows). The PCR reactions are performed using intact organisms from colonies that have been spotted from an agar plate containing the *E. coli* transformants harboring plasmids with genomic inserts to microtiter dishes. The presence of a PCR product in each reaction in the microtiter dish is determined by agarose gel electrophoresis. A BioMek 2000 workstation (Beckman Coulter, Fullerton, CA) capable of transferring portions of colonies to a microtiter dish for PCR reactions is used. The number of PCR products to be used is determined by our ability to obtain and assay for the presence of productive PCR products. With a dedicated thermocycler running full time, 192 reactions per day can be prepared and checked by electrophoresis, and within 3 months 11,520 reactions can occur. Given the insert sizes, this represents 0.7 coverage of the genome, over 5000 open reading frames based on an estimate of 8000 in the genome (B.B Magee, Cold Spring Harbor, Sept. 1999). The number of genes in regulatory networks of other fungi suggests that multiple genes are isolated using this strategy.

A mini-array membrane spotted with PCR products is then be hybridized with radiolabeled cDNAs as described above. To avoid artifacts inherent in the use of different membranes and different spotting efficiencies, the same membrane is used for multiple labeled cDNA preparations by stripping the membrane between uses. The ability to strip with hot SDS solutions to remove at least 97.5% of the signal from individual spots and reuse nylon membranes is verified in control experiments using mini-arrays made from *S. cerevisiae* PCR products. Cox et al. To test that the membranes contain DNA that hybridize known genes, and to estimate the amount of the genome that is represented by the products on the membranes, hybridizations using ³³P-labeled DNA representing *HWP1, ACT1*, *ENO1* and other available genes is performed in control experiments. Additional control experiments consist of hybridization, stripping and rehybridization with the same probe, followed by PhosphorImager analysis to determine the intensity ratios of the same spot in two different hybridizations with the same probe. The ratio should be close to 1 for all spots. Once membranes are judged suitable, radiolabeled cDNA from *C. albicans* strains with or without the DNABPG described above are used in standard hybridization reactions.

Intensities of signals at each spot is determined with ImageQuant software and ratios determined for each spot for the different labeled cDNA species. The intensity ratio of spots with *HWP1* DNA serves as a standard for the level of difference that would be expected for other genes regulated in a similar manner and to verify that the design successfully identifies genes controlled by the DNABPG. The ratios of differences in intensities, and the direction of the difference, i.e. increased or decreased for DNABPG null mutant cDNA compared to isogenic strains with the gene is tabulated. If the DNABPG is important for expression of a given hypha-specific gene, either as an activator or as a repressor, the level of mRNA for the hypha-specific gene is decreased or increased respectively in the null DNABP mutant compared to isogenic strains (including a complemented strain) harboring the DNABPG. Genes like *HWP1* that are targets of an activating DNABPG are represented by the black spot on the array hybridized with labeled cDNA from strains having the DNABPG but not from strains lacking this gene.

To further verify that the expression of DNA in the spots is controlled by the DNABPG(s), Northern blots are performed as stated above except that the PCR product used to produce the spot (newly identified target gene) is used to prepare the probe for Northern blotting comparing blots with RNA from the isogenic strains as described.

The identity of the target genes controlled by the DNABPG is determined by retrieving the *E. coli* colony harboring the plasmid used to generate the PCR product that produced different signals with the differentially expressed cDNA. The DNA sequence of the plasmid insert is determined by dideoxynucleotide sequencing and used to search the *C. albicans* genome for the corresponding genes.

The genes are analyzed by standard homology searches in order to gain insight into the functional category of the gene product, cell wall protein, metabolism, signal transduction, secretion etc. Upstream regions of these newly identified genes are analyzed for the presence of the UAS or URS, or of similar sequences that might control the strength of binding of the DNABP. The identity of the genes forms the bases of new hypotheses about pathogenic mechanisms that will be directly testable in proposed future experiments. These experiments have a very high probability of revealing a global regulatory network that *C. albicans* deploys to produce a set of genes that permit invasion and proliferation in host tissues.

Mini and microarray approaches to pathogenic questions will be extremely important for advancing knowledge about *C. albicans* because of the present inability to employ random mutagenesis approaches with this diploid organism. Furthermore, the availability of membranes and/or chips for *C. albicans* is imminent.

As an alternative approach to the mini-array scheme, differential screening of *C. albicans* cDNA libraries are performed. For identification of target proteins that are activated during germ tube induction, a lambda ZAP cDNA library prepared from germ tube mRNA is transferred to a nitrocellulose membrane that will be probed with ³³P-labeled first strand cDNA prepared from PolyA+ RNA from the isogenic strains with or without the DNABPGs that were grown in germ tube-inducing conditions as described for the mini-arrayed membranes above. For identification of target proteins that are repressed during yeast growth, a yeast-phase cDNA library is prepared using poly A+mRNA isolated from yeast forms groing at 37°C, the physiologically relevant temperature, in YNB. The library will be proped with ³³P-labeled first strand cDNA prepared from PolyA+ RNA from the isogenic strains with or without the DNABPGs that were grown as yeast's using the previously described conditions.

cDNA clones that display differing signal strengths depending on whether the probing cDNA was prepared from strains with or without the DNABPG, as assessed by autoradiographic signal intensities, is plaque-purified. As for the mini-array experiments, intensities of signals of each clone are determined with ImageQuant software and ratios determined for each clone for the different labeled cDNA species. The intensity ratio of clones with *HWP1* DNA serves as a standard for the level of difference that would be expected for other genes regulated in a similar manner and to verify that the design successfully identifies genes controlled by the DNABPG. The ratios of differences in intensities, and the direction of the difference, i.e., increased or decreased for DNABPG null mutant cDNA compared to isogenic strains with the gene is tabulated. If the DNABPG is important for expression of a given hypha-specific gene, either as an activator or as a repressor, the level of mRNA for the hypha-specific gene is decreased or increased respectively in the null DNABP mutant compared to isogenic strains (including a complemented strain) harboring the DNABPG.

## Claims

1. A method of identifying a polypeptide which modulates attachment of *C. albicans* to human tissue, the method comprising contacting in vitro a test polypeptide with an Upstream Activating Sequence ("UAS") region of the *HWP1* promoter of *C. albicans* of SEQ ID NO: 1 and determining whether the test polypeptide binds the UAS region, such a binding being an indication that the test polypeptide modulates attachment of *C. albicans* to human tissue.

2. The method of claim 1, wherein said UAS regions comprise a nitrogen catabolic enzyme regulatory protein ("NIT2") binding site.

3. The method of claim 2, wherein said NIT2 binding site is bound by a DNA binding protein containing a NIT2 DNA binding domain.

4. The method of claim 3, wherein said NIT2 DNA binding domain comprises the protein sequence set forth in SEQ ID NO: 3.

5. The method of claim 3, wherein the DNA binding domain of said DNA binding protein comprises the protein sequence set forth in SEQ ID NO: 2.

6. A DNA molecule comprising the sequence of the *HWP1* promoter of *C. albicans* of SEQ ID NO: 1 fused to a reporter gene.

7. The DNA molecule of claim 6, wherein the reporter gene is the GFP.

8. A vector comprising a DNA molecule of claim 6 or 7.

9. A recombinant host cell comprising a vector of claim 8.

10. The use of an Upstream Activating Sequence ("UAS") region of the *HWP1* promoter of *C. albicans* of SEQ ID NO: 1, or of a DNA molecule of claim 6 or 7, or of a vector of claim 8 or of a recombinant cell of claim 9, for the identification in vitro of a polypeptide which binds an Upstream Activating Sequence ("UAS") region of the *HWP1* promoter of *C. albicans,* such a binding being an indication that the polypeptide modulates attachment of *C. albicans* to human tissue.

## Patentansprüche

1. Verfahren zum Identifizieren eines Polypeptids, das die Anheftung von *C. albicans* an menschliches Gewebe moduliert, wobei das Verfahren das In-Kontakt-Bringen eines Testpolypeptids *in vitro* mit einer Upstream Activating Sequence ("UAS") - Region des *HWP1*-Promotors von *C. albicans* nach SEQ ID NO: 1 und das Bestimmen, ob das Testpolypeptid die UAS-Region bindet, umfasst, wobei ein derartiges Binden ein Anzeichen dafür ist, dass das Testpolypeptid die Anheftung von *C. albicans* an menschliches Gewebe moduliert.

2. Verfahren nach Anspruch 1, wobei die UAS-Regionen eine Bindungsstelle für ein regulatorisches Protein eines stickstoffabbauenden Enzyms ("NIT2") umfassen.

3. Verfahren nach Anspruch 2, wobei die NIT2-Bindungsstelle von einem DNA-Bindungsprotein gebunden wird, das eine NIT2 DNA-Bindungsdomäne enthält.

4. Verfahren nach Anspruch 3, wobei die NIT2 DNA-Bindungsdomäne die in SEQ ID NO: 3 dargelegte Proteinsequenz umfasst.

5. Verfahren nach Anspruch 3, wobei die DNA-Bindungsdomäne des DNA-Bindungsproteins die in SEQ ID NO: 2 dargelegte Proteinsequenz umfasst.

6. DNA-Molekül, umfassend die an ein Reportergen fusionierte Sequenz des *HWP1-*Promotors von *C. albicans* nach SEQ ID NO: 1.

7. DNA-Molekül nach Anspruch 6, wobei es sich bei dem Reportergen um GFP handelt.

8. Vektor, umfassend ein DNA-Molekül nach Anspruch 6 oder 7.

9. Rekombinante Wirtszelle, umfassend einen Vektor nach Anspruch 8.

10. Verwendung einer Upstream Activating Sequence ("UAS") - Region des *HWP1-*Promotors von *C. albicans* nach SEQ ID NO: 1 oder eines DNA-Moleküls nach Anspruch 6 oder 7 oder eines Vektors nach Anspruch 8 oder einer rekombinanten Zelle nach Anspruch 9 zur Identifizierung eines Polypeptids *in vitro,* das eine Upstream Activating Sequence ("UAS") - Region des *HWP1*-Promotors von *C. albicans* bindet, wobei ein derartiges Binden ein Anzeichen dafür ist, dass das Polypeptid die Anheftung von *C. albicans* an menschliches Gewebe moduliert.

## Revendications

1. Méthode pour identifier un polypeptide qui module l'attachement de C. albicans à du tissu humain, la méthode comprenant la mise en contact in vitro d'un polypeptide test avec une région Séquence Activatrice Amont ("UAS") du promoteur HWP1 de C. albicans de SEQ ID NO: 1, et déterminer si le polypeptide test lie la région UAS, une telle liaison étant une indication que le polypeptide test module l'attachement de C. albicans à du tissu humain.

2. Méthode selon la revendication 1, dans laquelle ladite région UAS comprend un site de liaison d'une protéine régulatrice d'enzyme du catabolisme de l'azote ("NIT2").

3. Méthode selon la revendication 2, dans laquelle ledit site de liaison de NIT2 est lié par une protéine liant l'ADN contenant un domaine de liaison à l'ADN de NIT2.

4. Méthode selon la revendication 3, dans laquelle ledit domaine de liaison à l'ADN de NIT2 comprend la séquence protéique présentée dans SEQ ID NO: 3.

5. Méthode selon la revendication 3, dans laquelle le domaine de liaison à l'ADN de ladite protéine liant l'ADN comprend la séquence protéique présentée dans SEQ ID NO: 2.

6. Molécule d'ADN comprenant la séquence du promoteur HWP1 de C. albicans de SEQ ID NO: 1 fusionnée à un gène rapporteur.

7. Molécule d'ADN selon la revendication 6, dans laquelle le gène rapporteur est la GFP.

8. Vecteur comprenant une molécule d'ADN selon la revendication 6 ou 7.

9. Cellule hôte recombinante comprenant un vecteur selon la revendication 8.

10. Utilisation d'une région Séquence Activatrice Amont ("UAS") du promoteur HWP1 de C. albicans de SEQ ID NO: 1, ou d'une molécule d'ADN selon la revendication 6 ou 7, ou d'un vecteur selon la revendication 8 ou d'une cellule hôte recombinante selon la revendication 9, pour l'identification in vitro d'un polypeptide qui lie une région Séquence Activatrice Amont ("UAS") du promoteur HWP1 de C. albicans, une telle liaison étant une indication que le polypeptide module l'attachement de C. albicans à du tissu humain.


### SEQUENCE LISTING

<110> SUNDSTROM, PAULA
<120> METHODS FOR ALTERING THE EXPRESSION OF HYPHAL-SPECIFIC GENES
<130> 23878.0007
<140> PCT/US00/32464
   <141> 2000-11-29
<150> 60/167,672
   <151> 1999-11-29
<160> 11
<170> PatentIn Ver. 2.1
<210> 1
   <211> 1470
   <212> DNA
   <213> Candida albicans
<400> 1
   ggatctttct ttttcatttc ccttaaaacc gatcaagaaa gaaagtggaa ataaagctat 60
   gataaatgtt gattttgtgt aattcaatca actaagcacg tttgacagtt aaaaagtacg 120
   ttgttgttgt cctcgtctcg tctaatttct gttgacgagg attaataaca agaaatacag 180
   gaaaccctcc aaaaaaaaaa ttttggacct tacacgcaca taaattgcgg ataaacttgc 240
   cataataaaa actctttgaa acatacgata tgttattctt ttcataactg gaatattttt 300
   gctttttttt aacattatga acaattgaaa aaaaaaggaa atgaaaaggt aagagttgcc 360
   taaccattga aaataatagg ctaaggtttt tcctgatgcg tttaactaaa aaggaaataa 420
   caaaagttat tagcgataac ctgcgtaagg tgtcaacaaa atatattttg cacgttagct 480
   ctatagaaaa tatacaaact aaatccttaa ggaatttcct ctatatataa taggaaatcc 540
   ctctcacagt gaactgaatt atccatctga attatcagtc cactaattcc atcaataaaa 600
   tagattagtg tattgttctc ttcagtacaa ttactaccat tatgcaatgc tagcttattg 660
   ttcataatta gccatgttgc acaccctaat tcgaacatta actgtatcca tatttttctt 720
   gtccttcttt gtttttttct aacaaaaatg ttccagaatt ttttaaaaaa tatttgaaaa 780
   aacacataac actttgagta tgataatatc aactattgac ttgttttgaa agtaaagaat 840
   caaatttttt tctaactcga ctaatgcact ttacatcaac tggatgttat ttgcatctac 900
   tactataagc tcaaacaaat tatctttcaa aaatgttata attaacaagt catctataat 960
   tctttggatc caaaaacaag gaattcggaa attctgacga taaatgtcga ctcacaattc 1020
   attgtaaaaa gggagagttt tggtaggctc ataatcgctt ataatgtacc tctaaagtaa 1080
   tctaaaacaa acacaacctt tctaaaacct ataataataa ccctaatggc tcacaaccgg 1140
   gataagttag ttagcccagc tgtttttttt ttgccttatt tttatgacta cattttgttt 1200
   cactttttgt tgcgacttta ataccgtttt tgcaacttct ctttgtatca cctgtatccg 1260
   ccttttttaa catagcaact cttgtaaagt ccctttcttt tcccactatt ttatcattct 1320
   tgaaatatgt aatcagaata gtttttcaaa aactataaat aacggtcaaa ataaccggct 1380
   attttcaatt tccattcaac ttgttttctc aacaatatca aacacaacag gaatctccta 1440
   tagtcactcg cttttagttt cgtcaatatg                                 1470
<210> 2
   <211> 50
   <212> PRT
   <213> Candida albicans
<400> 2
   Cys Thr Asn Cys Gly Thr Lys Thr Thr Pro Leu Trp Arg Arg Asn Pro
   1               5                   10                  15
   Gln Gly Gln Pro Leu Cys Asn Ala Cys Gly Leu Phe Leu Lys Leu His
               20                  25                  30
   Gly Val Val Arg Pro Leu Ser Leu Lys Thr Asp Val Ile Lys Lys Arg
           35                  40                  45
   Gln Arg
       50
<210> 3
   <211> 50
   <212> PRT
   <213> Candida albicans
<400> 3
   Cys Thr Asn Cys Phe Thr Gln Thr Thr Pro Leu Trp Arg Arg Asn Pro
   1               5                   10                  15
   Asp Gly Gln Pro Leu Cys Asn Ala Cys Gly Leu Phe Leu Lys Leu His
               20                  25                  30
   Gly Val Val Arg Pro Leu Ser Leu Lys Thr Asp Val Ile Lys Lys Arg
           35                  40                  45
   Asn Arg
       50
<210> 4
   <211> 1438
   <212> DNA
   <213> Candida albicans
<400> 4
   taattctaat aactgatact aagttttgtt cctttttttg ggatttcttt tttttctaat 60
   tttgattgtt tttcaatttt gggttttcaa tattattgac aagagtcatt ttattgaata 120
   tttgttttgt ttactacatt aaaggtgata ggtactttta gtttttaaaa attgttttgt 180
   tcaaattgtt tatctttttc ttcttcttct acttgctttg ttttctgttt tcggttcata 240
   gttgatagct tttaataaat accccttttt ttttacaata gttagttcta agcttattca 300
   gtggtttaat tggtagaaaa tgtgagctct gtagcttatg gtatcttcta tagcaatata 360
   tttaacttgg acatagttca ttattctgga cgcatgaagg tgcaaagtca aaaagtgaga 420
   atatgcaaag aggtaattag atttctgtcc tattaattaa caaaaaatct atatatagac 480
   tgcaatattt aatacccccc aagtttgatt ctataatcct tcgatttcta taaagatata 540
   accatgaatc atgagtaaat accaaataga ttaatagtag aatctgtatg gtcgtgtaaa 600
   gctgttcatt aaaaactatt gatttaaatt tccccttcct cgtaatctct ttttttttgg 660
   gggggaggat cagccgcaat aaaagattga aaattattaa acaaaacaac aaaacgggaa 720
   accggaaacc tgagaaaaag aaagaaagaa ggaaaacttt tgttaagata ttaaatttta 780
   cgaagcaaat ttaaaatcca tacttaacaa ttattgttat tacgtacttc aacagggaaa 840
   gaaaaagtta agattttaaa tctctttatt cctttctttc ttttattatt atttaccctt 900
   aatataaatg tcaaaacaag atccacctcc agattataca aataggacat cagataatta 960
   taacccagat acaactgata atcataatat tcctccacct ttcactactc atcctataga 1020
   ggttcatcca ccaccattct cctcttctac ttcacctaat atccgtgtgc cagcatattt 1080
   tcaaaatcaa actacttcag gatggacaat agtaataaat aatcgatttt ggactgatgg 1140
   attcaggata tttgtttccg aagatgcatc taataagttt gatgccttca aaaaaagtaa 1200
   aaatcccgaa ataatacaat tacaagaaca aggtattgga gtaccgttat ttaaagctgt 1260
   cacgtcatat attcccttag caacaaaatt tataacgttt agaagatatg tccctactaa 1320
   tttacatcca tttgatattg ataaagatta ttatgattat tgtattgtta aacggaaatt 1380
   acacgttgga tatgatagtt atatatttga atttactcct gatcgagaat tcggatcc 1438
<210> 5
   <211> 32
   <212> DNA
   <213> Artificial Sequence
<220>
   <223> Description of Artificial Sequence: Synthetic oligonucleotide
<400> 5
   ggctcgaggg accttacacg cacataaatt gc 32
<210> 6
   <211> 32
   <212> DNA
   <213> Artificial Sequence
<220>
   <223> Description of Artificial Sequence: Synthetic oligonucleotide
<400> 6
   ggctcgagca aaagttatta gcgataacct gc 32
<210> 7
   <211> 32
   <212> DNA
   <213> Artificial Sequence
<220>
   <223> Description of Artificial Sequence: Synthetic oligonucleotide
<400> 7
   ggctcgaggt gtattgttct cttcagtaca tt 32
<210> 8
   <211> 32
   <212> DNA
   <213> Artificial Sequence
<220>
   <223> Description of Artificial Sequence: Synthetic oligonucleotide
<400> 8
   ggctcgagct cgactaatcg actttacatc aa 32
<210> 9
   <211> 29
   <212> DNA
   <213> Artificial Sequence
<220>
   <223> Description of Artificial Sequence: Synthetic oligonucleotide
<400> 9
   ggctcgagat gtcgactcac aattcattg 29
<210> 10
   <211> 28
   <212> DNA
   <213> Artificial Sequence
<220>
   <223> Description of Artificial Sequence: Synthetic oligonucleotide
<400> 10
   ggctcgaggt tgcgacttta ataccgtt 28
<210> 11
   <211> 29
   <212> DNA
   <213> Artificial Sequence
<220>
   <223> Description of Artificial Sequence: Synthetic oligonucleotide
<400> 11
   ggctcgagca tagcaactct tgtaaagtc 29